# EUROPEAN PATENT SPECIFICATION

(11) **EP 1 175 388 B1**
(45) Date of publication and mention of the grant of the patent: **15.03.2006**
(21) Application number: 99918906.1
(22) Date of filing: 30.04.1999
(51) Int. Cl.: C07C 211/27, C07C 211/29, C07C 321/30, A61K 51/00, A61K 51/04

(54) **SPECT IMAGING AGENTS FOR SEROTONIN TRANSPORTERS**
SPECT ABBILDUNGSREAGENZIEN FÜR SEROTONINTRANSPORTER
AGENTS D'IMAGERIE DESTINES A LA TOMOGRAPHIE MONOPHOTONIQUE D'EMISSION POUR TRANSPORTEURS DE SEROTONINE

(43) Date of publication of application: 30.01.2002
(73) Proprietor: TRUSTEES OF THE UNIVERSITY OF PENNSYLVANIA, Philadelphia, PA 19104-3147 (US)
(72) Inventor: Kung, Hank, F., Wynnewood, PA 19096 (US)
(74) Representative: Cornish, Kristina Victoria Joy
(86) International application number: PCT/US1999/009344
(87) International publication number: WO 2000/066537

(56) References cited:
- EP-A- 0 402 097
- WO-A-92/19210
- WO-A-93/12080
- DE-C- 4 128 183
- US-A- 5 071 636
- US-A- 5 688 485
- US-A- 5 783 171
- OYA S. ET AL.: "A new single-photon emission computed tomography imaging agent for serotonin transporters: (123 I) IDAM, 5-iodo-2-((2-((dimethylamino)methyl)-pheny l)thio)benzyl alcohol" JOURNAL OF MEDICINAL CHEMISTRY., vol. 42, no. 3, - 11 February 1999 (1999-02-11) pages 333-335, XP002238697 AMERICAN CHEMICAL SOCIETY. WASHINGTON., US ISSN: 0022-2623
- DATABASE CAPLUS ON STN CHEM. ABSTRACTS, (COLUMBUS OHIO, USA) 74:22490 FRETER K. ET AL.: 'New group of anorexigenic compounds' & J. MED. CHEM. vol. 13, no. 6, June 1970, pages 1228 - 1230
- DATABASE CAPLUS ON STN CHEM. ABSTRACTS, (COLUMBUS OHIO, USA) 114:228508 MEHTA N. B. ET AL.: 'Preparation of diphenyl sulfides as nervous system agents' & EP 402 097 A

## Description

Part of the work performed during development of this invention utilized U.S. Government funds under NIH Grant No. NS-35120. The U.S. Government has certain rights in this invention.

This invention relates to novel compounds for CNS neurotransmitter systems, especially for the neurotransmitter serotonin, which can be utilized to image neurotransmitter reuptake systems in the brain.

Depression, with its related conditions, is one of the most common mental disorders in the United States. It is estimated that about five percent of the adult population experiences a depressive episode in their lifetime that requires antidepressive, drug therapy. A chemical in the human brain, called serotonin, has been linked with depression and with other psychiatric disorders such as eating disorders, alcoholism, pain, anxiety and obsessive-compulsive behavior.

Serotonin (5-HT) is an essential neurotransmitter for the normal function of the central nervous system. This neurotransmission system in the brain controls various important behaviors, including sleep awake cycle, mood, temperature, appetite, etc. In addition, several commonly used anti-anxiety drugs (Frazer, A. and J. G. Hensler, *Ann. NY Acad. Sci. 600*:460-475 (1990); Gozlan, H. and M. Hamon, *Anxiety: Neurobiol., Clinic and Ther. Persp. 232*:141-150 (1993)) and antidepressants (Frazer, A., *J. Clin. Psychiatry 6*:9-25 (1997); Coryell, W., *J. Clin. Psychiatry 1*:22-27 (1998); Heninger, G. R. *et al., Pharmacopsychiatry 29(1)*:2-11 (1996); Fuller, R. W., *Prog. Drug Res. 45*:167-204 (1995)) interact specifically with serotonin neurotransmission. Pharmacological actions of the antidepressants (selective serotonin reuptake inhibitors; SSRI), such as fluoxetine (Wong, D. T. and F. P. Byrnaster, *Biology 363*:77-95 (1995)), paroxetine (Holliday, S. M. and G. L. Plosker, *Drugs Aging 3(3)*278-299 (1993)) and sertraline (Lasne, M. C. *et al., Int. J. Rad Appl. Inst. - Part A, Applied Rad Isot. 40(2)*:147-151 (1989)), are based on blockade of presynaptic transporters for serotonin. Thus, studies of radioligand binding to serotonin transporter (SERT) may provide valuable information of these sites in normal and various disease states. Several tritiated ligands including imipramine (Raisman, R. *et al., Eur. J. Pharmacol. 54*:307-308 (1979)), citalopram (D'Amato, *R. et al., Pharmacol. Exp. Ther. 242*(*l*):364-371 (1987)), paroxetine (Habert, E., *et al., Eur. J. Pharmacol. 118*(*1-2*):107-114 (1985)) and 6-nitroquipazine (Hashimoto, K., and T. Goromaru, *Biochem. Pharmacol. 41(11)*:1679-1682 (1991); Hashimoto.K, and T. Goromaru, *Neuropharmacology 30(2)*:113-117 (1991)) have been used for *in vitro and in vivo* studies. A reduced level of SERT labeled by these tritiated ligands has been demonstrated in post mortem brain sections of patients with depression (Perry, E. K*. et al., Br. J. Psychiat. 142*:188-192 (1983)), Alzheimer's and Parkinson's diseases (D'Amato, R. *et al., Pharmacol. Exp. Ther. 242(1)*:364-371 (1987)) as well as in the frontal cortex of a suicide victim (Mann, J. J., *Nature Medicine 4(1*):25-30 (1998)). The *in vitro* binding studies suggest that using *in vivo* imaging methods to evaluate the density of SERT may be clinically important.

Anti-depressive drugs, such as Prozac, operate to inhibit serotonin reuptake by binding with the serotonin transporter (SERT) protein, effectively blocking the binding of the protein with serotonin. Although Prozac has been found to be an effective anti-depressant treatment, it has side effects which can be serious. Prozac is known to bind to the serotonin transporter (SERT) protein, but the responses of patients can differ widely. Some patients experience greater binding than others. If a patient is not responding to Prozac treatment, there is currently no way to determine why that is the case. Frequently, the physician will simply administer greater doses of the drug, a practice which will not necessarily lead to better results and which can enhance undesirable side effects.

Development of selective tracers for positron emission tomography (PET) and single photon emission tomography (SPECT) have made it possible to study *in vivo* neuroreceptors or site-specific bindings non-invasively in the human brain. However, development of PET or SPECT tracers specifically for *in vivo* imaging of SERT has only met with limited success. The most promising radioligand described to date is [¹¹C](+)McN5652 for PET imaging (Szabo, Z. *et al., Synapse 20(1)*:37-43 (1995); Szabo, Z. *et al., J. Nucl. Med 37(5)*:125 (1996); Szabo, Z. *Behav. Brain Res. 73(1)*:221-224 (1995); Szabo, *Z. et al., J. Cerebral Blood Flow & Metabol. 15(5)*:798-805 (1995); Suehiro, M. *et al., J. Nucl. Med. 34(1)*:120-127 (1993); Suehiro, M. *et al., Nucl. Med Biol. 22(4)*:543-545 (1995)). Specific binding of [¹¹C](+)McN5652 correlates well with the known density of SERT sites in the human brain (Szabo, Z. *et al., Synapse 20(1):*37-43 (1995)). In search of a clinically useful SPECT ligand for SERT, several radioiodinated compounds have been evaluated including 4-I-tomoxetine (Kung, M. P. *et al., Life Sci. 51*:95-106 (1992)). Among these tracers only [¹²⁵I]5-iodo-6-nitroquipazine (Biegon, A. *et al., Brain Res. 619*:236-246 (1993); Mathis. C. A. *et al., Brain Res. 619*:229-235 (1993); Mathis, C. A. *et al., Eur. J. PharmacoL 210(1)*:103-104 (1992)) showed promising properties for mapping SERT sites in monkey's brain (Jagust, W. J. *et al., J. Nucl. Med. 37(7)*:1207-1214 (1996)). No human study of [¹²³I]5-iodo-6-nitroquipazine has been reported. The high nonspecific binding and the fast peripheral metabolism observed with [¹²³I]-5-iodo-6-nitroquipazine in non-human primates may limit its application as a clinically useful SPECT imaging agent for SERT in the human brain. Previously, it has been suggested that [¹²³I]β-CIT(2β-carbomethoxy-30-(4-iodophenyl)tropane), a SPECT imaging agent, which binds to both DAT and SERT, will be able to clarify pathological changes in both dopaminergic and serotonergic systems. However, overlapping uptake regions and differential kinetics of [¹²³I]β-CIT binding to DAT and SERT were observed (Fujita, M. *et al., Eur. J. Nucl. Med. 23(4)*:431-436 (1996); Kuikka, J. T. *et al., Eur. J. Nucl. Med 22(4)*:346-250 (1995); Tiihonen, J. *et. al., Eur. J. Nucl. Med. 24(10)*:1253-1260 (1997)). Nonetheless, the effect of a selective SSRI in human brain *in vivo* has been directly measured by [¹²³I]β-CIT/SPECT imaging of SERT sites in depressed patients undergoing treatment with citalopram (Pirker, W. *et al., J. Neural Trans. Gen. Sec. 100(3)*:247-256 (1995)). A more selective series of compounds, nor-β-CIT (N-demethylated analog of β-CIT) (Bergstrom, K. A. *et al., Eur. J. Nucl. Med. 24(6)*:596-601 (1997)) and related derivatives, (Blough, B. E*. et al., J. Med Chem. 40(24)*:3861-3864 (1997)) have recently been reported as improved SPECT imaging agents for SERT. It is suggested that [¹²³I]nor-β-CIT might be a suitable alternative tracer for visualization of SERT sites in the human brain with SPECT (Bergstrom, K. A. *et al. Eur. J. NucL Med 24(6)*:596-601 (1997); Hiltunen. J. *et al., Eur. J. Nucl. Med 25(1)*:19-23 (1998)). However, [¹²³I]nor-β-CIT still binds to both DAT and SERT, and the selectivity is not sufficient to distinguish between these two motioamline transporter sites. The need for a selective SERT/SPECT imaging agent is still unfulfilled. Therefore, there is a strong impetus to find an improved agent with a better selectivity for imaging SERT in the brain.

A chlorinated compound, 5-chloro-2-((2-((dimethyl-amino)methyl)phenyl)thio)benzyl alcohol (403U76), was reported as an inhibitor for serotonin uptake and norepinephrine uptake in rat brain synaptosomes (Kᵢ = 2.1 and 55 nM, respectively) (Ferris, R. M. *et al., J. Pharm. Pharmacol. 47*:775-781 (1995); Brieaddy, L. E., *"Substituted diphenylsulfides as serotonin uptake inhibitors,"* published International Patent Appl. No. WO 93/12080 (1993); Mehta, N. B. *et al., "Halogen substituted diphenylsulfides,"* published European Patent Application EP 402,097 Al (1990)).

Oya S. *et al J. Med. Chem. 1999, 42(3)333-5* describes preliminary work including data on SERT binding and imaging of brominated, iodinated and radioiodinated species of one specific type of compound, 1,5-halo-2-((2-((dimethylamino)methyl)-phenyl)thio)benzy] alcohol. The compounds are benzyl alcohol derivatives, i.e., each compound must contain a hydroxymethyl group directly attached to a ring. This reference does not specifically teach or suggest any other types of compounds.

A first aspect of the present invention is directed to compounds of Formula ***I*:** 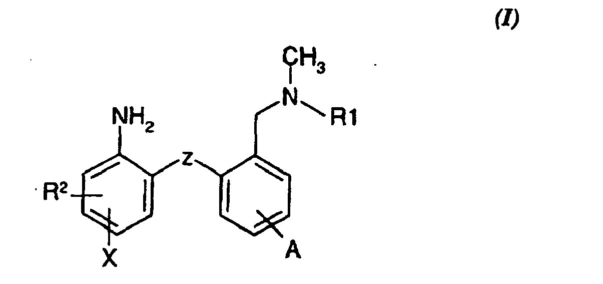
or a pharmaceutically acceptable salt thereof; wherein
X is hydrogen, Cl, Br, I, NO₂, NR³R⁴ or -L-Ch;
Z is S, O, NR⁶, CR⁷R⁸, C(O) or -C(=CR⁷R⁸)-;
A is hydrogen, Cl, I, Br or -L-Ch;
R¹ is hydrogen, C₁-C₅ alkyl, C₃-C₈ cycloalkyl, C₁₋₅ alkylcarbonyl, (C₃₋C₈ cycloalkyl)carbonyl, phenyl, benzyl, naphthyl, naphthylmethyl or -L-Ch;
R² is hydrogen or methyl;
R³ and R⁴ are independently hydrogen, hydroxy, C₁-C₅ alkyl, C₃-C₈ cycloalkyl, C₁₋₅ alkylcarbonyl, (C₃-C₈ cycloalkyl)carbonyl, phenyl, benzyl, naphthyl, naphthylmethyl or -L-Ch;
R⁶ is hydrogen, C₁-C₅ alkyl, C₃-C₈ cycloalkyl, C₁₋₅ alkylcarbonyl, (C₃₋C₈ cycloalkyl)carbonyl, phenyl, benzyl, naphthyl, or naphthylmethyl or -L-Ch;
R⁷ and R⁸ are independently hydrogen, C₁-C₅ alkyl or chloro;
L is a covalent bond or a linking group selected from the group consisting of-(CH₂)ₙ-, or -(CH₂)ₙ-C(O)-, where n is 1-5; and
Ch is a tetradentate ligand capable of chelating a metal;
with the proviso that one and only one of A, X, R¹,R³,R⁴ or R⁶ is -L-Ch; or with the proviso that X or A is Br or I.

The present invention is further directed to radiolabeled compounds of Formula ***I*** wherein X or A is radioactive iodine or radioactive bromine.

The present invention is further directed to radiolabeled compounds (or intermediate thereof) of Formula ***I*** wherein one of X, R¹, R³, R⁴, R⁵ or R⁶ is-L-Ch.

The present invention is also directed to imaging agents comprising a compound of claim 1 where X is a radioactive bromine or iodine isotope.

The present invention is also directed to imaging agents comprising a compound of claim 1 where one of X, R¹, R³, R⁴, R⁵ or R⁶ is -L-Ch, and Ch is one of Formulae ***V***, ***VI*** or ***VII***.

### Brief Description of the Figures

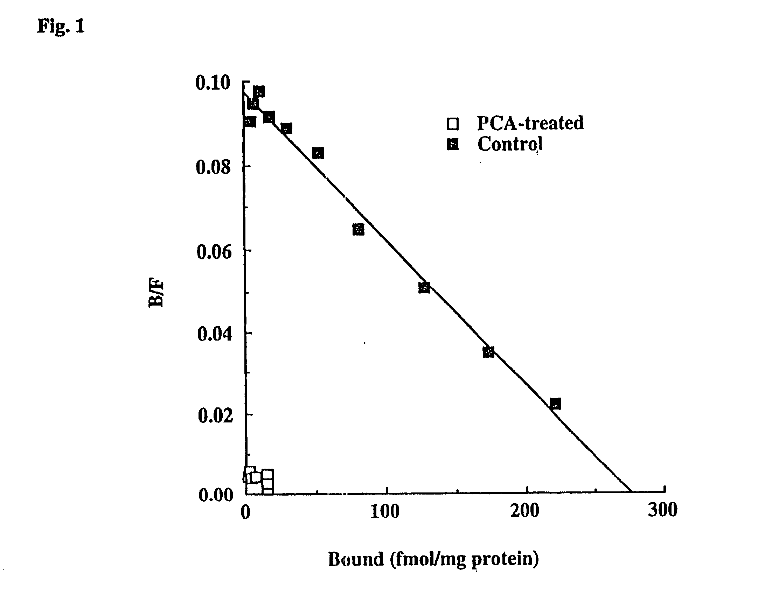

FIG. 1 depicts a Scatchard analysis of [¹²⁵I]IDAM binding to frontal cortical membrand homogenate prepared from saline control and PCA-treated rats. The data are the average values from 4 control and 4 PCA-treated rats (Control: K_{d} = 0.25 nM ± 0.05 nM, Bₘₐₓ = 272 ± 30 fmol/mg protein PCA-lesion : K_{d} = 0.050 ±0.15 nM, Bₘₐₓ, = 20 ± 7 fmol/mg protein).

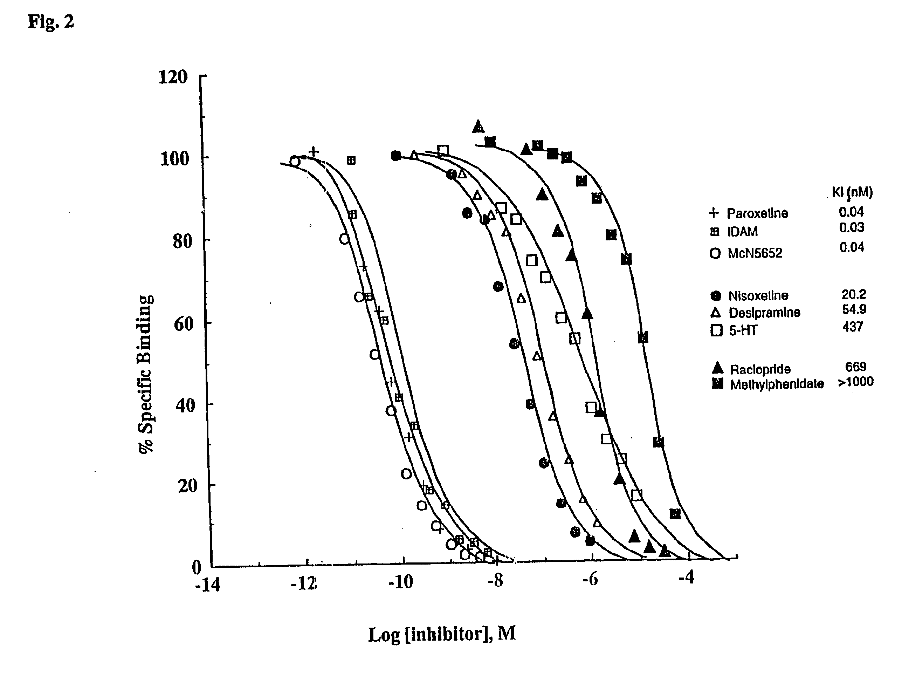

FIG. 2 plots the potencies of compounds to inhibit the specific binding of [¹²⁵I]IDAM to membranes prepared from rat frontal cortical homogenate. Mean values of triplicate determinations are shown in the graph from three independent experiments, each performed in duplicate.

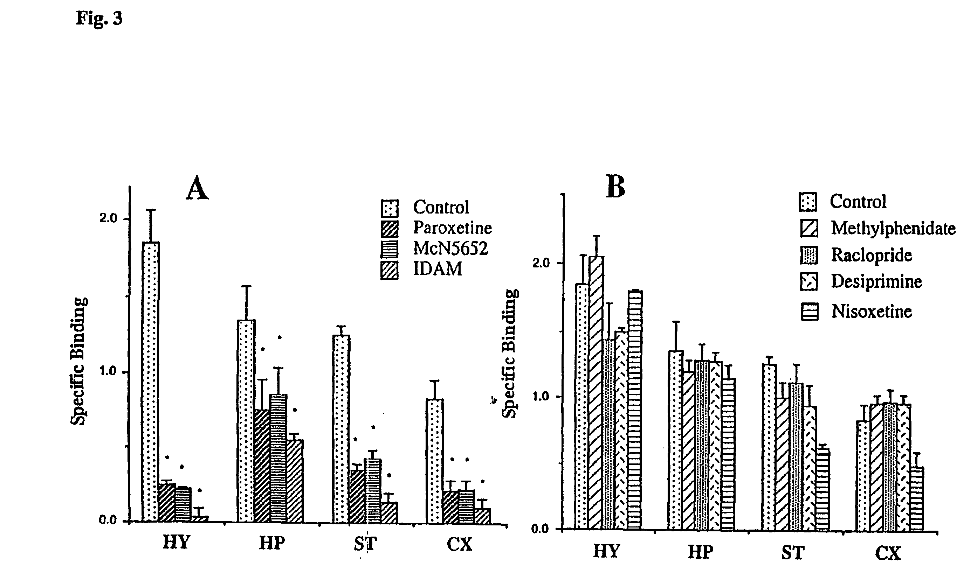

FIGs. 3A and 3B depict the effect of pretreatment with various compounds on specific binding of [¹²⁵I]IDAM in rat brain regions. Rats were pretreated with various drugs at the dose of 2 mg/kg, i.v. 5 min prior to the tracer administration. Sixty minutes after the tracer injection, specific binding in each brain region was compared between saline-pretreated (control) and drug-pretreated rats. Values are presented as the average ± SD of three rats in each point * p<0.05.

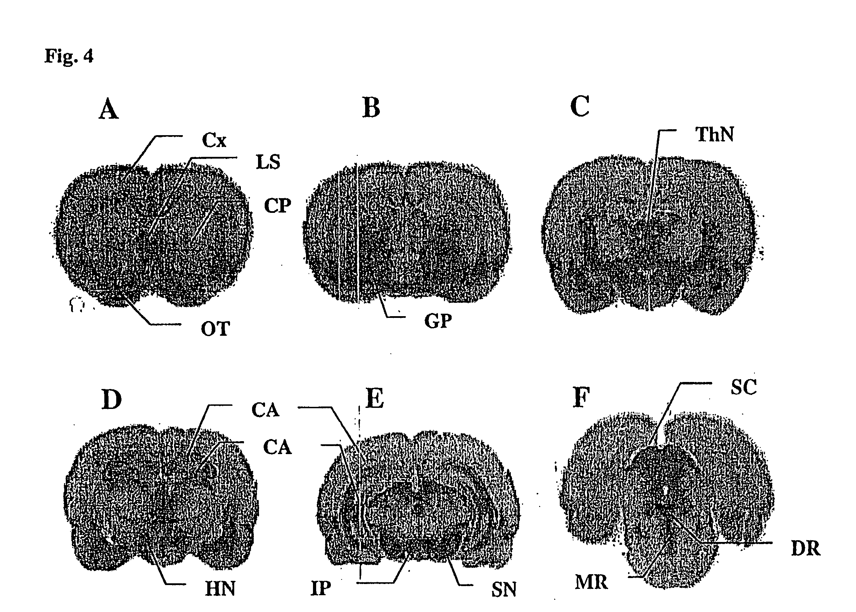

FIGs. 4A - 4F depict *ex vivo* autoradiographic localization of [¹²⁵I]IDAM binding sites in rats after 60 min post-injection. High levels of radioactivity were observed in the areas containing high densities of serotonin transporter sites. The coronal sections corresponding to a stereotaxic atlas are as follows: 4A: plate 14; 4B: plate 19; 4C: plate 24; 4D: plate 31; 4E: plate 39; 4F: plate 48. CX: cortex; Cpu: caudate putamen; LSD: lateral septum; OT: olfactory tubercle; GP: globus pallidus; ThN: thalamus nuclei; HN: hypothalamic nuclei; CA1, CA2: fields CA1, CA2 Ammon's hom; IP: interpeduncular nucleus; SN: substantia nigra; SC: superior colliculus; DR: dorsal raphe; MR: medial raphe.

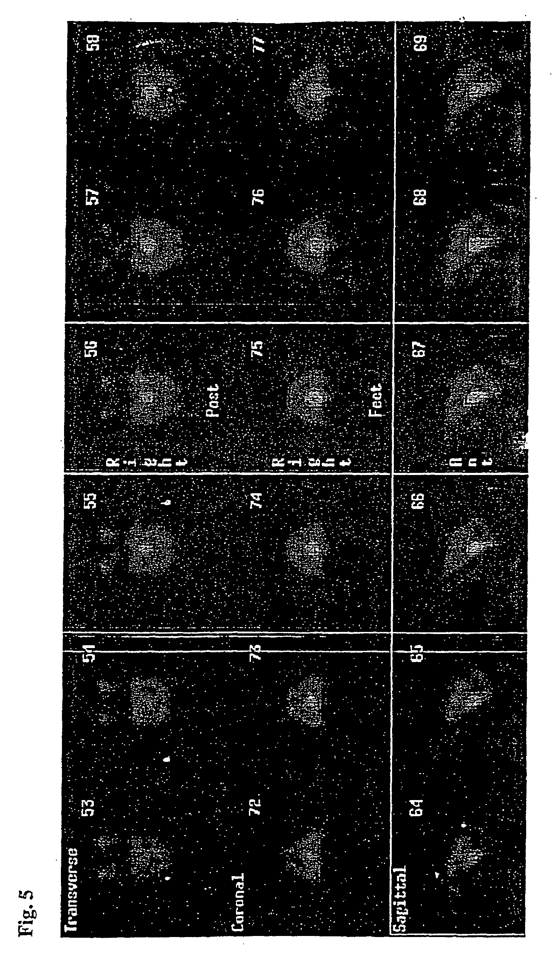

FIG. 5 depicts transverse, coronal and saggital views of SPECT (upper row) images of the baboon head. The SPECT image represents the summed counts acquired 60-120 min after injection of [¹²³I]IDAM. A high concentration of activity was localized in the midbrain area (MB: raphe nucleus, substantia nigra and hypothalamus) where SERT is highly concentrated

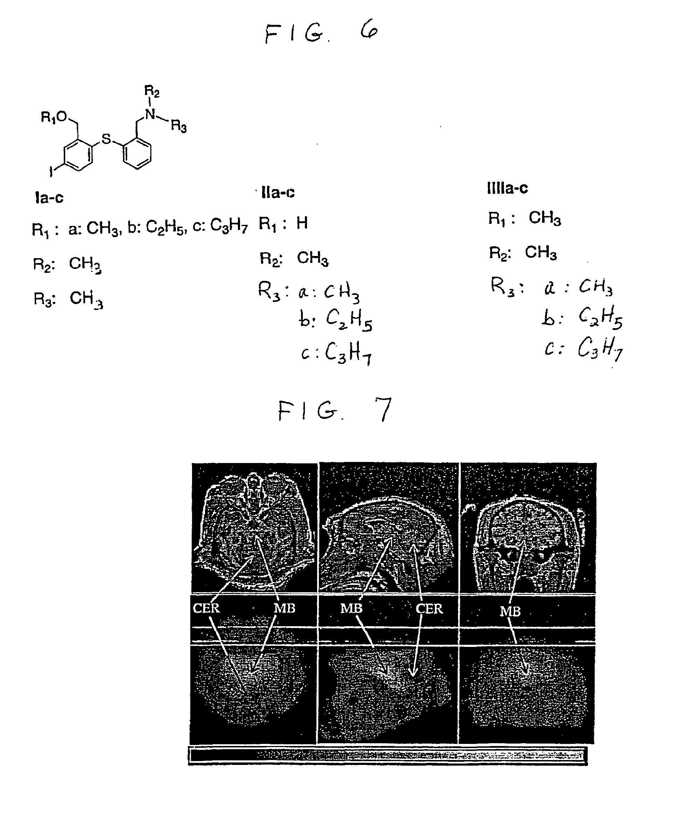

FIG. 6 lists the structures of various compounds that can be synthesized according to the methods described in the application.
FIG. 7 depicts a comparison of MRI images (anatomy) and SPECT images of [¹²³I]IDAM (SERT localization), presented in transaxial, sagittal and coronal views (between 60-120 minutes post-i.v. injection). [¹²³I]IDAM localized with high concentration in midbrain (MB, raphe nucleus, substantia nigra, hypothalamus) where the SERT concentration is high; it displayed no specific uptake in cerebellum (CER) an area lacking SERT. The ratio of MB/CER uptake at 120 minutes was 2.4.

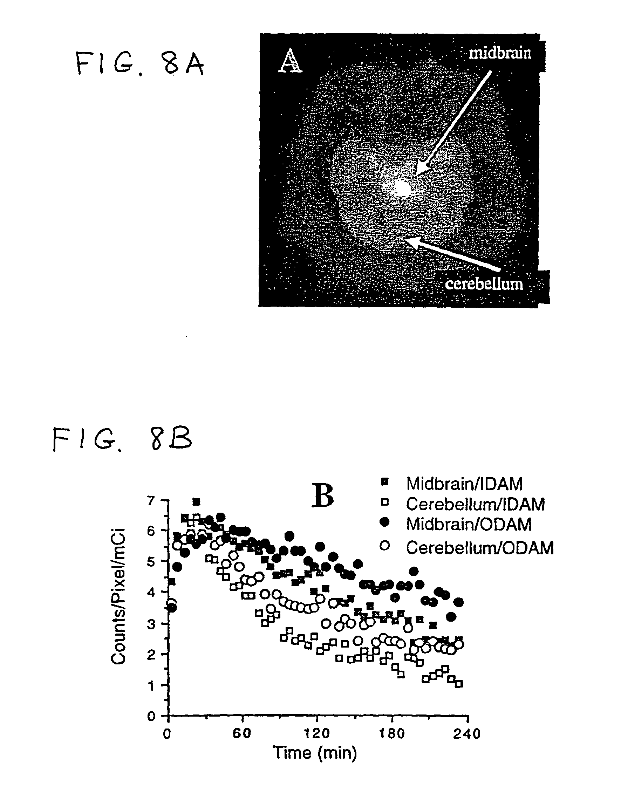

FIG. 8A depicts a summed transverse image at the point of pseudo-equilibrium (approx. 90-120 min post-injection) approximately at the level of the midbrain. FIG. 8B depicts time-activity curves for the midbrain and cerebellum for both [¹²³I]IDAM and [¹²³I]ODAM (both in the same baboon).

### Detailed Description of the Invention

A first aspect of the present invention is directed to compounds of Formula ***I*:** 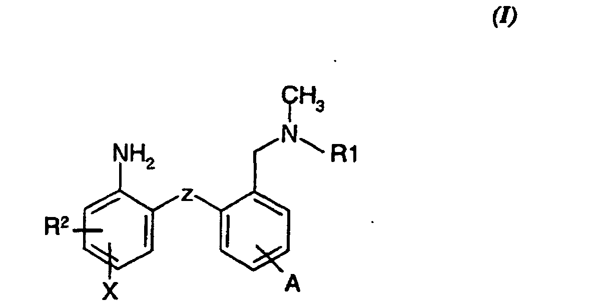
or a pharmaceutically acceptable salt thereof; wherein
X is hydrogen, Cl, Br, I, NO₂, NR³R⁴ or -L-Ch;
Z is S, O, NR⁶, CR⁷R⁸, C(O) or -C(=CR⁷R⁸)-;
A is hydrogen, Cl, I, Br or -L-Ch;
R¹ is hydrogen, C₁-C₅ alkyl, C₃-C₈ cycloalkyl, C₁₋₅ alkylcarbonyl, (C₃₋C₈ cycloalkyl)carbonyl, phenyl, benzyl, naphthyl, naphthylmethyl or -L-Ch;
R² is hydrogen or methyl;
R³ and R⁴ are independently hydrogen, hydroxy, C₁-C₅ alkyl, C₃-C₈ cycloalkyl, C₁₋₅ alkylearbonyl, (C₃-C₈ cycloalkyl)carbonyl, phenyl, benzyl, naphthyl, naphthylmethyl or -L-Ch;
R⁶ is hydrogen, C₁-C₅ alkyl, C₃-C₈ cycloalkyl, C₁₋₅ alkylcarbonyl, (C₃₋C₈ cycloalkyl)carbonyl, phenyl, benzyl, naphthyl, or naphthylmethyl or -L-Ch;
R⁷ and R⁸ are independently hydrogen, C₁-C₅ alkyl or chloro;
L is a covalent bond or a linking group selected from the group consisting of-(CH₂)ₙ-, or -(CH₂)ₙ-C(O)-, where n is 1-5; and
Ch is a tetradentate ligand capable of chelating a metal;
with the proviso that one and only one of A, X, R¹, R³, R⁴ or R⁶ is -L-Ch; or with the proviso that X or A is Br or I.

The compounds of the present invention may have the general Formula I, wherein:
X is Br or I;
Z is S, O, NR⁶, CR⁷R⁸, C(O) or-C(=CR⁷R⁸)-;
A is hydrogen;
R¹ is hydrogen, C₁-C₅ alkyl, C₃-C₈ cycloalkyl, C₁₋₅ alkylcarbonyl, (C₃₋C₈ cycloalkyl)carbonyl, phenyl, benzyl, naphthyl or naphthyhnethyl;
R² is hydrogen or methyl;
R⁶ is hydrogen, C₁-C₅ alkyl, C₃-C₈ cycloalkyl, C₁₋₅ alkylcarbonyl, (C₃₋C₈ cycloalkyl)carbonyl, phenyl, benzyl, naphthyl or naphthyltnethyl; and
R⁷ and R⁸ are independently hydrogen, C₁-C₅ alkyl or chloro.

The compounds of the present invention may have the general Formula I, wherein:
X is hydrogen; Cl, Br, I, NO₂ or NR³R⁴;
Z is S, O, NR⁶, CR⁷R⁸, C(O) or -C(=CR⁷R⁸)-;
A is Br or I;
R¹ is hydrogen, C₁-C₅ alkyl, C₃-C₈ cycloallcyl, C₁₋₅ alkylcarbonyl, (C₃₋C₈ cycloalkyl)carbonyl, phenyl, benzyl, naphthyl or naphthylmethyl;
R² is hydrogen or methyl;
R³ and R⁴ are independently hydrogen, hydroxy, C₁-C₅ alkyl, C₃-C₈ cycloalkyl, C₁₋₅ alkylcarbonyl, (C₃-C₈ cycloalkyl)carbonyl, phenyl, benzyl, naphthyl or naphthylmethyl;
R⁶ is hydrogen, C₁-C₅ alkyl, C₃-C₈ cycloalkyl, C₁₋₅ alkylcarbonyl, (C₃₋C₈ cycloalkyl)carbonyl, phenyl, benzyl, naphthyl or naphthylmethyl; and
R⁷ and R⁸ are independently hydrogen, C₁-C₅ alkyl or chloro.

The present invention is further directed to radiolabeled compounds of Formula *I* wherein X or A is radioactive iodine or radioactive bromine.

X may be a radioactive isotope selected from the group consisting of ¹²³I, ¹³¹I, ¹²⁵I, ⁷⁷Br and ⁷⁶Br.

A may be a radioactive isotope selected from the group consisting of ¹²³I, ¹³¹I, ¹²⁵I, ⁷⁷Br and ⁷⁶Br.

The compounds of the invention may have the general formula: 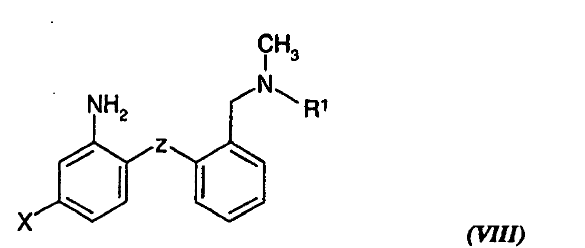
or 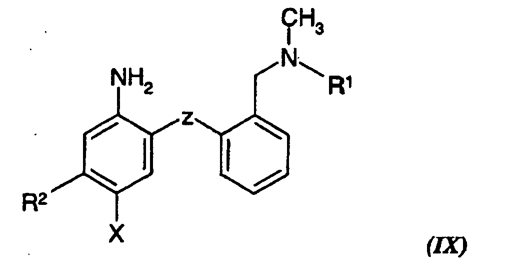
or a pharmaceutically acceptable salt thereof, where X, Z, R¹ and R² are as defined above for Formula ***I**.*

The compounds of the invention may have the general formula: 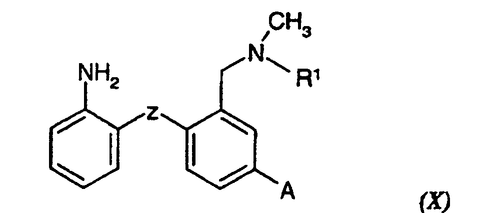
or a pharmaceutically acceptable salt thereof, where
A is Br, I or -L-Ch; where L and Ch are as defined above for Formula ***I***;
Z is O, S, N(CH₃), C(O), C(=CH₂), or CH₂, preferably O, S or CH₂; and
R¹ is as defined above for Formula ***I***.

A may be ¹²³I, ¹³¹I, ¹²⁵I, ⁷⁷Br, or ⁷⁶Br; or A may be -L-Ch, where Ch is Formula ***II*** or Formula ***V*** and L is a covalent bond or C₁₋₂ alkylene group.

Preferred compounds of Formula ***VIII*** include compounds, or pharmaceutically acceptable salts thereof, where
X is I or Br,
Z is S, O, N(CH₃), C(O), C(=CH₂), or CH₂, more preferably S; and
R¹ is methyl.
X may be ¹²³I, ¹³¹I, ¹²⁵I, ⁷⁷Br or ⁷⁶Br, most preferably ¹²³I.

Alternatively, compounds of Formula VIII may include compounds, or pharmaceutically acceptable salt thereof, wherein:
X is selected from the group consisting of I, ¹²³I, ¹³¹I, and ¹²⁵I;
Z is -S-; and
R¹ is methyl.

Preferred compounds of Formula *IX* include compounds, or pharmaceutically acceptable salts thereof, where
X is hydrogen, I or Br,
Z is S; and
R¹ is hydrogen or methyl.
X may be ¹²³I, ¹³¹I, ¹²⁵I, ⁷⁷Br or ⁷⁶Br.

In the compounds of the present invention, Ch may be selected from the group consisting of: 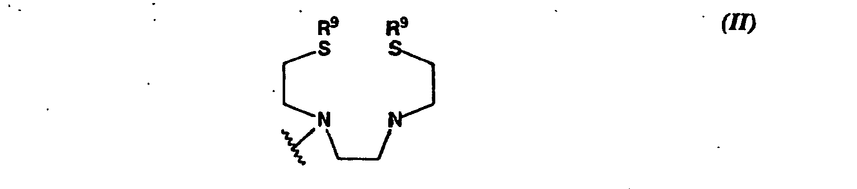 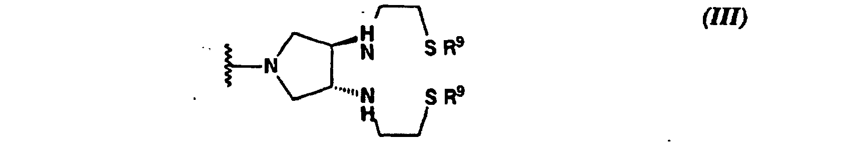 and 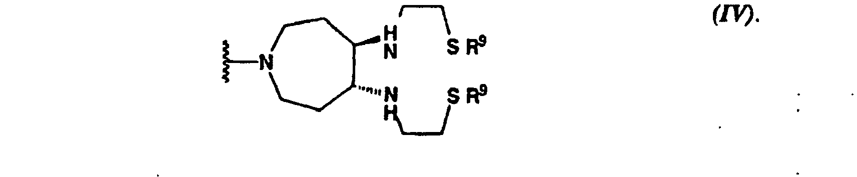
wherein R⁹ is a sulphydryl protecting group, which may be hydrogen, methoxymethyl, merhoxyethoxyroethyl, *p*-methoxybenzyl or benzyl.

The present invention also provides a radiopharmaceutical complex, comprising a compound as defined in claim 14, and a radioactive metal selected from the group consisting of technetium-99m, rhenium-186 and rhenium-188 coordinated therewith.

The present invention further provides a technetium chelate of a compound of the first aspect of the invention, wherein Ch is selected from the group consisting of: 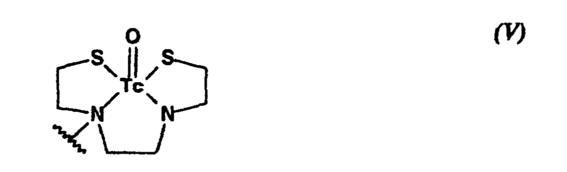 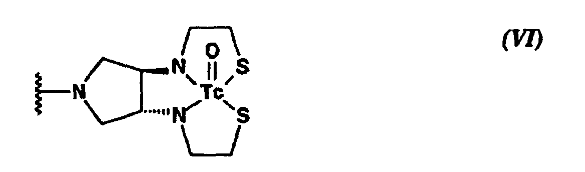 and

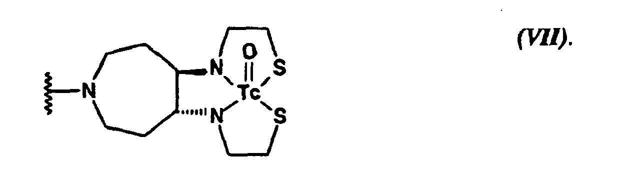

In this aspect, compounds having Ch ligands, such as those of Formulae II, III and IV are complexed with 99m-pertechnetate, as described herein to form metal chelates.

Compounds of the present invention include compounds (6), (7), (13)-(16) below, as well as compounds K109, K99026, K99028 in Tables 1A and 1B: 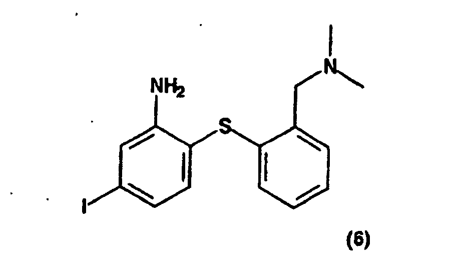 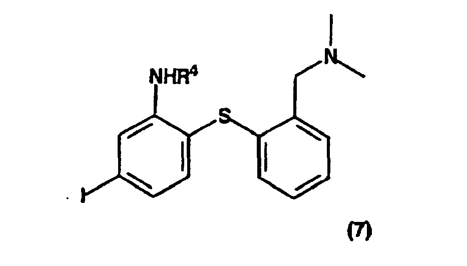 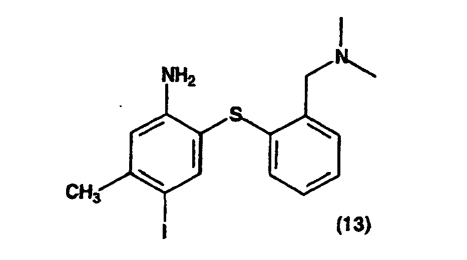 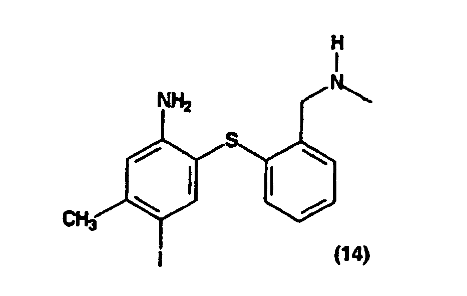 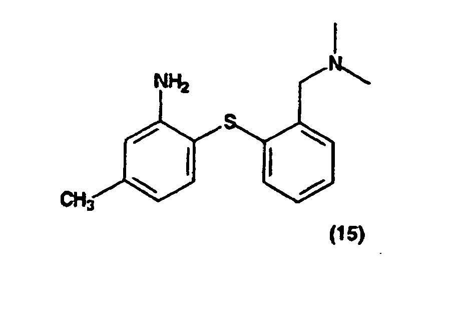 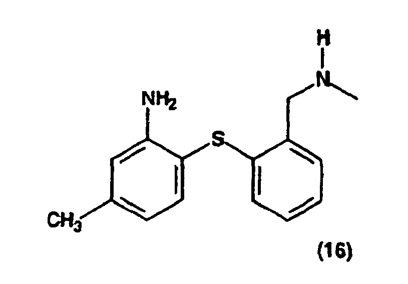
as well as acid addition salts thereof, where
R⁴ is defined above; and
each I is preferably ¹²³I. ¹³¹I, or ¹²⁵I, and each Br is preferably ⁷⁷Br or ⁷⁶Br.

The compounds (1) - (5), (8) - (12) and (17)- (20) below and the compounds listed in Tables 1A and 1B, other than K109, K99026 and K99028, FIG. 6 and the target compounds of Schemes 9-13 are presented not as embodiments of the invention, but as examples useful for understanding the invention. 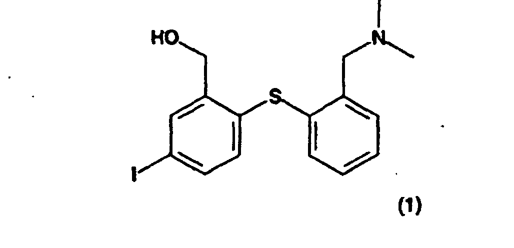 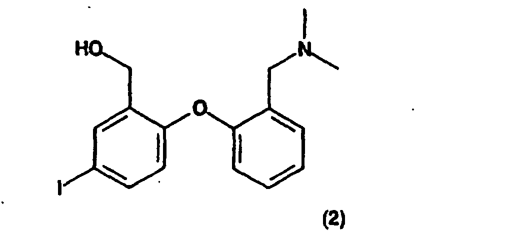 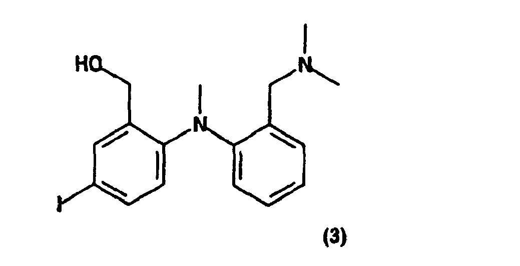 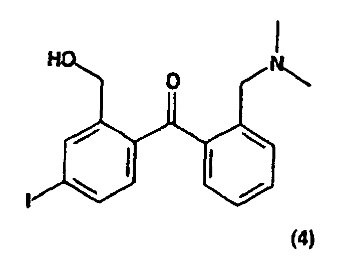 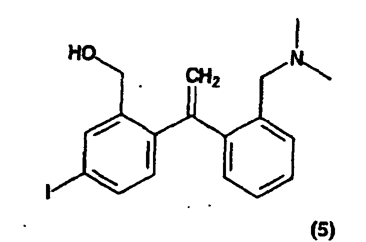 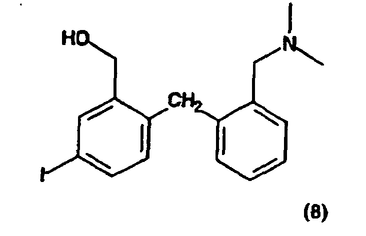 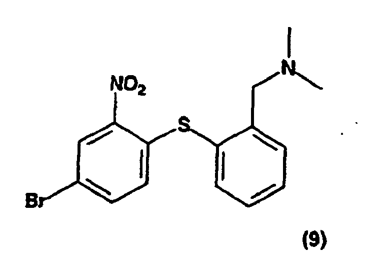 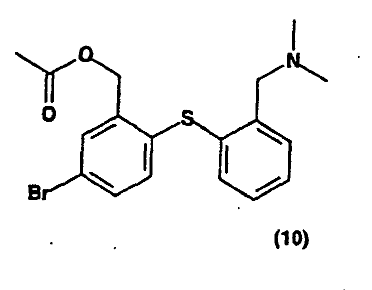 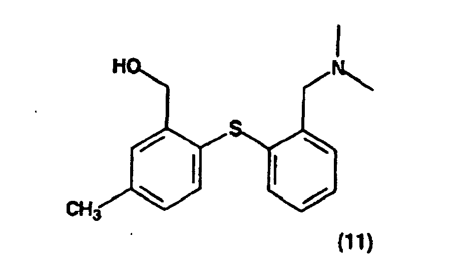 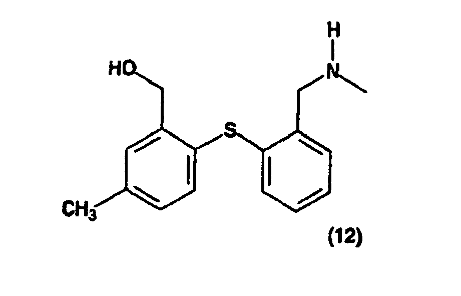 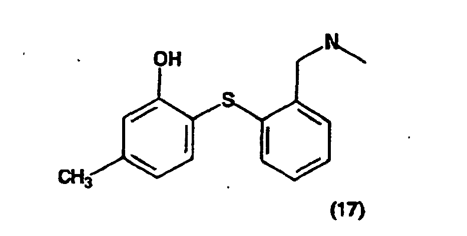 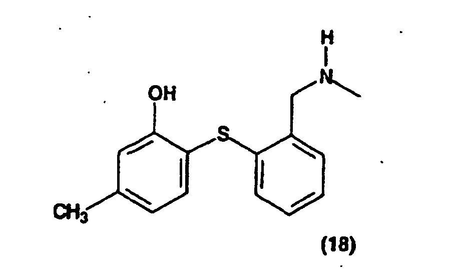 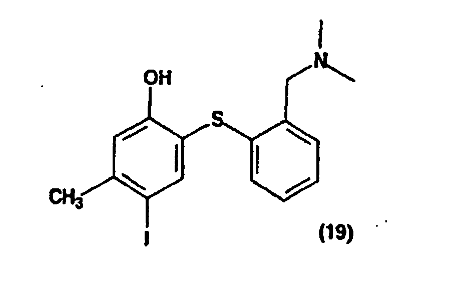 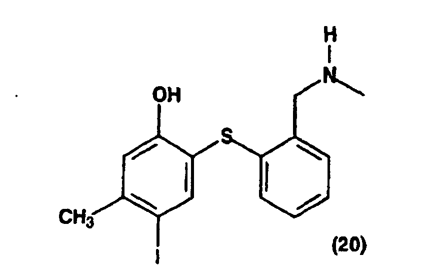
as well as acid addition salts thereof, where
R⁴ is defined above; and
each I is preferably ¹²³I, ¹³¹I or ¹²⁵I, and each Br is preferably ⁷⁷Br or ⁷⁶Br.

When the compounds of this invention are to be used as imaging agents, they must be labeled with suitable radioactive halogen isotopes. Although ¹²⁵I-isotopes are useful for laboratory testing, they will generally not be useful for actual diagnostic purposes because of the relatively long half-life (60 days) and low gamma-emission (30-65 Kev) of ¹²⁵I. The isotope ¹²³I has a half life of thirteen hours and gamma energy of 159 KeV, and it is therefore expected that labeling of ligands to be used for diagnostic purposes would be with this isotope. Other isotopes which may be used include ¹³¹I (half life of 2 hours). Suitable bromine isotopes include ⁷⁷Br and ⁷⁶Br.

The radiohalogenated compounds of this invention lend themselves easily to formation from materials which could be provided to users in kits. Kits for forming the imaging agents can contain, for example, a vial containing a physiologically suitable solution of an intermediate of Formula I in a concentration and at a pH suitable for optimal complexing conditions. The user would add to the vial an appropriate quantity of the radioisotope, e.g., Na¹²³I, an oxidant, such as hydrogen peroxide. The resulting labeled ligand may then be administered intravenously to a patient, and receptors in the brain imaged by means of measuring the gamma ray or photo emissions therefrom.

Pharmaceutically-acceptable salts of the compounds of this invention include the acid addition salts derived from non-toxic inorganic acids such as hydrochloric acid, nitric acid, phosphoric acid, sulfuric acid, hydrobromic acid, hydroiodic acid, phosphorous acid and the like. Also included are those salts derived from non-toxic organic acids such as aliphatic mono and dicarboxylic acids, for example acetic acid, phenyl-substituted alkanoic acids, hydroxy alkanoic and alkanedioic acids, aromatic acids, and aliphatic and aromatic sulfonic acids.

Examples of useful organic acids are carboxylic acids such as maleic acid, acetic acid, tartaric acid, propionic acid, fumaric acid, isethionic acid, succinic acid, cyclamic acid, pivalic acid and the like, useful inorganic acids are hydrohalide acids such as HCl, HBr, HI; sulfuric acid; phosphoric acid and the like. Preferred acids for forming acid addition salts include HCl and acetic acid.

The term "alkyl" as employed herein by itself or as part of another group refers to both straight and branched chain radicals of up to 8 carbons, such as methyl, ethyl, propyl, isopropyl, butyl, *t*-butyl, isobutyl, pentyl, hexyl, isohexyl, heptyl, and octyl.

In embodiments where one of X, R¹, R³, R⁴, R⁵ or R⁶ is -L-Ch, the groups R⁹ are both hydrogen, or can be any of the variety of protecting groups available for sulfur, including methoxymethyl, methoxyethoxymethyl, *p-*methoxybenzyl or benzyl. Sulfur protecting groups are described in detail in Greene, T.W. and Wuts, P.G.M., *Protective Groups in Organic Synthesis,* 2nd Edition, John Wiley and Sons, Inc., New York (1991). Protecting group P^{a} can be removed by appropriate methods well known in the art of organic synthesis, such as trifluoroacetic acid, mercuric chloride or sodium in liquid ammonia. In the case of Lewis acid labile groups, including acetamidomethyl and benzamidomethyl, R⁹ can be left intact. Labeling of the ligand with technetium in this case will cleave the protecting group, rendering the protected diaminedithiol equivalent to the unprotected form.

Tc-99m complexes are prepared as follows. A small amount of non-radiolabeled compound (1-2 mg) is dissolved in 100 *µ*L EtOH and mixed with 200 *µ*L. HCl (1 N) and 1 mL Sn-glucoheptonate solution (containing 8-32 *µ*g SnCl₂ and 80-320 *µ*g Na-glucoheptonate, pH 6.67) and 50 *µ*L EDTA solution (0.1 N). [^{99m}Tc]Pertechnetate (100-200 *µ*L; ranging from 2-20 mCi) saline solution are then added. The reaction is heated for 30 min at 100°C, then cooled to room temperature. The reaction mixture is analyzed on TLC (EtOH:conc. NH₃ 9:1) for product formation and purity check. The mixture can be neutralized with phosphate buffer to pH 5.0.

The radioactive diagnostic agent should have sufficient radioactivity and radioactivity concentration which can assure reliable diagnosis. For instance, in case of the radioactive metal being technetium-99m, it may be included usually in an amount of 0.1 to 50 mCi in about 0.5 to 5.0 ml at the time of administration. The amount of a compound of Formula *I* may be such as sufficient to form a stable chelate compound with the radioactive metal.

The thus formed chelate compound as a radioactive diagnostic agent is sufficiently stable, and therefore it may be immediately administered as such or stored until its use. When desired, the radioactive diagnostic agent may contain any additive such as pH controlling agents (e.g., acids, bases, buffers), stabilizers (e.g., ascorbic acid) or isotonizing agents (e.g., sodium chloride).

The present invention further relates to a method of preparing a technetium-99m complex according to the present invention by reacting technetium-99m in the form of a pertechnetate in the presence of a reducing agent and optionally a suitable chelator with an appropriate Ch-containing compound.

The reducing agent serves to reduce the Tc-99m pertechnetate which is eluted from a molybdenum-technetium generator in a physiological saline solution. Suitable reducing agents are, for example, dithionite, formamidine sulphinic acid, diaminoethane disulphinate or suitable metallic reducing agents such as Sn(II). Fe(II), Cu(I). Ti(III) or Sb(III). Sn(II) has proven to be particularly suitable.

For the above-mentioned complex-forming reaction, technetium-99m is reacted with an appropriate compound of the invention as a salt or in the form of technetium bound to comparatively weak chelators. In the latter case the desired technetium-99m complex is formed by ligand exchange. Examples of suitable chelators for the radionuclide are dicarboxylic acids, such as oxalic acid, malonic acid, succinic acid, maleic acid, orthophtalic acid, malic acid, lactic acid, tartaric acid, citric acid, ascorbic acid, salicylic acid or derivatives of these acids; phosphorus compounds such as pyrophosphates; or enolates. Citric acid, tartaric acid, ascorbic acid, glucoheptonic acid or a derivative thereof are particularly suitable chelators for this purpose, because a chelate of technetium-99m with one of these chelators undergoes the desired ligand exchange particularly easily.

The most commonly used procedure for preparing [Tc^{v}O]⁺³N₂S₂ complexes is based on stannous (II) chloride reduction of [^{99m}Tc]pertechnetate, the common starting material. The labeling procedure normally relies on a Tc-99m ligand exchange reaction between Tc-99m (Sn)-glucoheptonate and the N₂S₂ ligand. Preparation of stannous (II) chloride and preserving it in a consistent stannous (II) form is critically important for the success of the labeling reaction. To stabilize the air-sensitive stannous ion it is a common practice in nuclear medicine to use a lyophilized kit, in which the stannous ion is in a lyophilized powder form mixed with an excess amount of glucoheptonate under an inert gas like nitrogen or argon. The preparation of the lyophilized stannous chloride/sodium glucoheptonate kits ensures that the labeling reaction is reproducible and predictable. The N₂S₂ ligands are usually air-sensitive (thiols are easily oxidized by air) and there are subsequent reactions which lead to decomposition of the ligands. The most convenient and predictable method to preserve the ligands is to produce lyophilized kits containing 100-500 *µ*g of the ligands under argon or nitrogen.

Since the radiopharmaceutical composition according to the present invention can be prepared easily and simply, the preparation can be carried out readily by the user. Therefore, the present invention also relates to a kit, comprising:
(1) A non-radiolabeled compound of the invention, the compound optionally being in a dry condition; and also optionally having an inert, pharmaceutically acceptable carrier and/or auxiliary substances added thereto; and
(2) a reducing agent and optionally a chelator,

wherein ingredients (I) and (2) may optionally be combined; and
further wherein instructions for use with a prescription for carrying out the above-described method by reacting ingredients (1) and (2) with technetium-99m in the form of a pertechnetate solution may be optionally included.

Examples of suitable reducing agents and chelators for the above kit have been listed above. The pertechnetate solution can be obtained by the user from a molybdenum-technetium generator. Such generators are available in a number of institutions that perform radiodiagnostic procedures. As noted above the ingredients (1) and (2) may be combined, provided they are compatible. Such a monocomponent kit, in which the combined ingredients are preferably lyophilized, is excellently suitable to be reacted by the user with the pertechnetate solution in a simple manner.

A further aspect of the present invention provides a process of radioimagining, comprising:
(i) administering to a mammal an effective amount of a radiopharmaceutical composition, wherein said composition comprises: a radiopharmaceutical compound of claim 4 and a pharmaceutically acceptable carrier or diluent, and
(ii) radioimaging said mammal after allowing sufficient time for said composition to localize in a tissue of said mammal.

A yet further aspect of the present invention provides a process of radioimaging, comprising:
(i) administering to a mammal an effective amount of a radiopharmaceutical composition, wherein said composition comprises: a radiopharmaceutical compound of claim 6 and a pharmaceutically acceptable carrier or diluent, and
(ii) radioimaging said mammal after allowing sufficient time for said composition to localize in a tissue of said mammal.

A still further aspect of the present invention provides a process of radioimaging, comprising:
(i) administering to a mammal an effective amount of a radiopharmaceutical composition, wherein said composition comprises: a technetium compound of claim 16 and a pharmaceutically acceptable carrier or diluent; and
(ii) radioimaging said mammal after allowing sufficient time for said composition to localize in a tissue of said mammal.

A further aspect of the present invention provides a process of radioimaging, comprising:
(i) administering to a mammal an effective amount of a composition, wherein said composition comprises a radiopharmaceutical compound of claim 13 and a pharmaceutically acceptable carrier or diluent, and
(ii) radioimaging said mammal after allowing sufficient time for said composition to localize in a tissue of said mammal.

### Methods of Making

Diphenylsulfide compounds of the present invention can be synthesized using methods analogous to those described in Ferris, R. M. *et al., J. Pharm. Pharmacol.* 47:775-781 (1995); Brieaddy, L. E., *"Substituted diphenylsulfides as serotonin uptake inhibitors,"* published International Patent Appl. No. WO 93/12080 (1993); and Mehta, N. B. *et al., "Halogen substituted diphenylsulfides,"* published European Patent Application EP 402,097 A1 (1990).

Additionally, compounds of the invention can be formed by the following methods, illustrated in the schemes appearing at the end of the detailed description. Schemes 1-4, 7-13 are provided not as embodiments of the invention, but as examples useful for understanding the invention.

Scheme 1 depicts a synthetic method for forming IDAM.

Scheme 2 depicts a synthetic method for forming compounds where Y is -CH₂ OR⁵ and R⁵ is various lower alkyl groups.

Scheme 3 depicts a synthetic method for forming compounds where R¹ can be various lower alkyl groups.

Scheme 4 depicts a synthetic method for preparing compounds where Z is oxygen, such as ODAM.

Schemes 5 and 6 depict synthetic routes for preparing compounds of the invention where Y is -NR³ R⁴.

Scheme 7 depicts a synthetic route for preparing compounds where Y is -L-Ch, where L is a bond and Ch is Formula ***II**.*

Scheme 8 depicts synthetic steps for preparing compounds having various groups at the R¹ position.

Schemes 9, 10 and 11 depict a synthetic method for forming compounds where Z is -CH₂-, A is iodo and Y is hydrogen, dimethylaminomethyl or hydroxymethyl, such as CARDAM1, CARDAM2 and CARDAM5X.

Scheme 12 depicts a synthetic method for forming compounds where Z is NH, and one of A or X is iodo.

Scheme 13 depicts a synthetic method for preparing compounds where X is - L-Ch, where L is a bond and Ch is Formula ***II*** or ***V***.

Experimental Proton NMR spectra were run on a Brukker 200 spectrometers. The chemical shifts (δ) were reported in ppm downfield from the tetramethylsilane standard; CDCl₃ or CD₃OD was used as the solvent. Mass spectra were carried out at Department of Chemistry, University of Pennsylvania.

### Example 1

### 5-iodo-2-[[2-2[(dimethylamino)methyl]phenyl]thio]benzyl alcohol (IDAM) and (5-bromo-2-[[2-2](dimethylamino)methyl]phenyllthio]benzyl alcohol)

The synthesis of 5-iodo-2-[[2-2((dimethylamino)-methyl]phenyl]thio]benzyl alcohol (IDAM) and its bromo derivative (5-bromo-2-[[2-2[(dimethylamino)methyl]phenyl]thio]benzyl alcohol) was achieved by a reaction sequence outlined in Scheme 1 shown on page 37 of this application. The direct coupling of 2,5-dibromobenzoic acid (Frazer, A., *J Clin. Psychiatry,* 6:9-25 (1997)) or 2.5-diiodobenzoic acid (Mathis, C.A. *et al., J Nucl. Med.* 33:890(1992)) with 2-thio-*N*,*N*-dimethylbenzamide (Wong, D.T. *et al., Adv. Exp. Med. & Biol.,* 363:77-95 (1995)) was carried out in *N*,*N*-dimethylacetamide (DMAC) with sodium methoxide to give the desired compounds in good yield (59 and 44% for 23 and 28, respectively). Only when 2-thio-*N.N-*dimethylbenzamide was freshly prepared, was a good coupling yield achieved. This may due to the fact that the free thiol of 22 was not stable upon prolonged standing at room temperature. The bromo compound was converted to the corresponding tri-*n*-butyltin derivative (Maryanoff. E.M. *et al., J Med Chem.* 33:2793-2797 (1990)) by a tetrakis(triphyenlphosphine)palladium(0)-catalyzed reaction with good yield (66%). The tin derivative was successfully converted to IDAM with excellent yield (97%), or alternatively, 2-((4-iodo-2-carboxyphenyl)thio)-*N*,*N* dimethylbenzamide (Mathis, C.A. *et al., Eur. J. Pharmacol. 210*:103-104 (1992)) was reduced to IDAM with 66% yield.

### Example 2

### Radioiodination of 5-iodo-2-[[2-2-[(dimethylamino)methyl]phenyl]thio]benzyl alcohol

Radioiodination was carried out by an iododestannylation reaction (Maryanoff, E.M. *et al., J. Med. Chem.* 33:2793-2797 (1990)). IDAM was treated with bistributyltin and Pd(PPh₃)₄ (see Example 4, Step (I)). The stannylated intermediate was reacted with radioactive sodium iodide (I-125 or I-123) in the presence of hydrogen peroxide. The final [¹²⁵I or ¹²³I]IDAM was purified using HPLC method (purity>99%, Rt=11.38 min, PRP-1 column eluted, 1 mL/min, with a 80:20 mixture of acetonitrile and 3,3-dimethylglutaric acid buffer, pH 7.4).

The radioiodinated ligand, [¹²⁵I] or [¹²³I]IDAM (26, Scheme 1), was successfully prepared from the corresponding tributyltin derivative by an iododestannylation reaction, which resulted in a nocarrier-added tracer (specific activity is comparable to that of Na¹²⁵I; or Na¹²³I) . The radiochemical identity of the radioiodinated ligand was verified by a co-injection of the nonradioactive compound. IDAM. which showed an identical retention time of 7 min on HPLC profiles. [¹²⁵I]IDAM has been shown to be stable in 50% ethanol for up to two months after radioiodination (> 95% radiochemical purity determined by HPLC). [¹²³I]IDAM displayed excellent *in vitro* stability with no decomposition observed upon prolonged standing (>20 hr) at 0-4°C in saline solution.

### Example 3

### 5-Iodo-2-(2-dimethylaminomethylphenoxy)benzyl alcohol

**Methyl, 5-nitro-2-(2-methylphenoxy)-benzoic acid (34)** To a solution of 2-chloro-5-nitro benzoic acid 31 (4.04 g, 20 mmol) in nitrobenzene (20 mL) was added K₂CO₃ (4.14 g, 3 eq) followed by Cu (0.2 g) and Cul (0.2 g) at 70-80°C. The mixture was stirred at 80°C for 10 min. O-Cresol (4.32g, 2 eq) was added and the mixture was stirred at 155 °C overnight. NaOH solution (1M, 30 mL) was added after the mixture was cooled down and the dark slurry was extracted with ether to remove nitrobenzene. The aqueous phase was filtered and acidified with HCl. The resulting mixture was extracted with mixed solvent (CH₂Cl₂:MeOH=9:1). The organic phase was dried, filtered and concentrated to give a tar (4.6 g). To the solution of tar obtained above in MeOH (100 mL) was added concentrated H₂SO₄ (2 mL) dropwise at room temperature and the mixture was stirred under reflux overnight. Solvent was removed and ice water was added. The mixture was made basic with KOH solution and extracted with CH₂Cl₂. The organic phase was dried, filtered and concentrated to give crude product which was purified by chromatography (Flash 40) (EtOAc:Hex=1:10) to give 720 mg of product and 2.5 g of mixture (product and by product). ¹H NMR (CDCl₃, δ): 2.19 (s, 3H, ArCH₂), 3.96 (s, 3H, COOCH₃), 6.73 (d, 1H, J=9.2 Hz, ArH), 7.00 (d,d,, 1H, J=7.4, 1.8 Hz, ArH), 7.15-7.34 (m, 3H, ArH), 8.23 (d,d, 1H, J=7.5, 1.8 Hz, ArH), 8.79 (d, 1H, J=2.8 Hz, ArH).

**Methyl, 5-nitro-2-(2-bromomethylphenoxy)-benzoic acid (35)** To a solution of starting material **34** (720 mg, 2.5 mmol) in CCl₄ (20 mL) was added NBS (491 mg, 1 eq) and AIBN (40 mg, 0.24 mmol). The mixture was stirred under reflux overnight. The cool solution was filtered and the solvent was removed to give an oil which was purified by chromatography (Flash 40) (EtOAc:Hex=1:10) to give 130 mg of product and 710 mg of mixture (product and byproduct). ¹H NMR (CDCl₃, δ): 3.94 (s, 3H, COO(CH₃), 4.56 (s. 2H, NCH₂), 6.95 (d, 2H, J=9.1 Hz, ArH), 7.24 (t,d, 1H, J=7.4, 1.1 Hz, ArH, 7.36 (t, d, 1H, J=7.6, 1.7 Hz, ArH), 7.52 (d,d, 1H, J=7.4, 1.7 Hz, ArH), 8.25 (d,d, 1H, J=9.2, 2.8 Hz, ArH), 8.81 (d, 1H, J=2.8, 1.1 Hz, ArH).

**Methyl, 5-nitro-2-(2-dimethylaminomethylphenoxy)-benzoic acid (36)** To a solution of starting material 35 (300 mg, 0.82 mmol) in acetonitrile (10 mL) was added diemthylamine (5 mL, 2M in THF) and triethylamine (1 mL) dropwise at room temperature. The mixture was stirred under reflux overnight. Solvent was removed and the residue was purified by preparative thin layer chromatography (PTLC) (CH₂Cl₂:MeOH=9:1) to give 148 mg of product. ¹H NMR (CDCl₃, δ): 2.15 (s, 6H, NCH₃), 3.39 (s, 2H, NCH₂), 3.92 (s, 3H, COOCH₃), 6.73 (d, 1H, J=9.2 Hz, ArH), 6.97 (d, 1H, J=7.5 Hz, ArH), 7.24 (t,d, 1H, J=7.0, 1.1 Hz, ArH), 7.30 (t,d, 1H, J=7.3, 1.7 Hz, ArH), 7.52 (d, 1H, J=7.5 Hz, ArH), 8.16 (d,d,d, 1H, J=9.2, 2.8, 1.1 Hz, ArH, 8.75 (d,d, 1H, J=2.8,1.0 Hz, ArH), HRMS Calcd(C₁₇H₁₈N₂O₅): M/Z 331.1294 (M⁺+1); Found: M/Z 331.1301 (M⁺+1).

**Methyl, 5-amino-2-(2-dimethylaminomethylphenoxy)-benzoic acid (37)** A mixture of starting material 36 (225 mg, 0.68 mmol) and Pd/C (50 mg) in mixed solvent (30 mL, MeOH:EtOAc=1:9) was hydrogenated at 50 psi and room temperature for 2 h. The mixture was filtered and the filtrate was concentrated to give crude product which was purified by PTLC (Ch₂Cl₂:MeOH=9:1) to give 220 mg of product. ¹H NMR (CDCL₃, δ): 2.67 (s, 6H, NCH₃), 3.70 (s, 3H, COOCH₃), 4.15 (s, 2H, NCH₂), 6.59 (d, 1H, J=8.1 Hz, ArH), 6.84 (d, 2H, J=1.6 Hz, ArH), 7.04 (t, 1H, J=7.4 Hz, ArH), 7.17 (d,d, 1H, J+7.7, 1.6 Hz, ArH), 7.24 (t, 1H, J=1.1 Hz, ArH), 7.64 (d,d, 1H, J=7.4, 1.5 Hz, ArH). HRMS Calcd(C₁₇H₂₀N₂O₃): M/Z301.1552 (M⁺+1); Found: M/Z301.1549 (M⁺+1).

**5-Amino-2-(2-dimethylaminomethylphenoxy)-benzyl alcohol (38)** To a suspension of LAH (240 mg, 6.3 mmol) in THF (20 mL) was added dropwise a solution of starting material **37** (410 mg. 1.4 mmol) at room temperature. The mixture was stirred at room temperature for 1 h. 0.2 mL of water, 0.2 mL of NaOH solution (1M) and 0.6 mL of water were added dropwise successively to quench the reaction. The mixture was filtered and washed with mixed solvent (CH₂Cl₂:MeOH=9:1). The filtrate was concentrated and the residue was purified by PTLC (CH₂Cl₂:MeOH:NH₄OH=9:1:0,1) to give 280 mg of product. ¹H NMR (CD₃OD, δ): 2.26 (s, 6H, NCH₃), 3.58 (s, 2H, NCH₂), 4.33 (s, 2H, HOCH₂), 6.58 (d,d, 1H, J+8.1, 1.0 Hz, ArH), 6.67 (d,d, 1H, J+8.5, 2.6 Hz, ArH), 6.74 (d, 1H, J=8.4 Hz, ArH), 6.82 (d, 1H, J=2.5 Hz, ArH), 6.96 (t,d, 1H, J=7.4,1.2 Hz, ArH), 7.14 (t,d, 1H, J=8.0, 1.8 Hz, ArH), 7.29 (d,d, 1H, J=7.4, 1.7 Hz, ArH), HRMS Calcd(C₁₆H₂₀N₂O₂): M/Z 273.1603 (M⁺+1); Found: M/Z 273.1603 (M⁺+1).

**5-Iodo-2-(2-dimethylaminomethylphenoxy)-benzyl alcohol (39)** To a mixture of starting material **38** (250 mg, 0.92 mmol), ice (3.6 g) and concetrated HCl (2.4 mL) was added a solution of NaNO₂ 9360 mg) in water (4.5 mL) dropwise at 0°C in an ice bath. The mixture was stirred at 0°C for 30 min. The resulting mixture was added into a solution of KI (2.1 g) in water (15 mL) dropwise at 0°C and the resulting mixture was stirred at room temperature for 30 min. The mixture was made basic with saturated NaHCO₃ solution and extracted with mixed solvent (CH₂CL₂:MeOH=9. 1). The organic phase was dried, filtered and concentrated to give crude product which was purified by PTLC (CH₂Cl₂:MeOH=9:1) to give 108 mg of product. ¹H NMR (CDCl₃, δ): 2.25 (s, 6H, NCH₃), 3.49 (s, 2H, NCH₂), 4.29 (s, 2H, HOCH₂), 6.67 (d, 1H, J=8.0 Hz, ArH), 6.77 (d, 1H, J=8.2 Hz, ArH), 7.02 (t, 1H, J=7.5 Hz, ArH), 7.19 (d,d, 1H, J=7.9, 1.2 Hz, ArH), 7.24 (d, I H, J=7.2 Hz, ArH), 7.59 (d,d, 1H, J=8.4, 1.7 Hz, ArH), 7.63 (s, 1H, ArH), HRMS Calcd(C₁₆H₁₈INO₂): M/Z 384.0461 (M⁺+1); Found M/Z 384.0472 (M⁺+1).

### Example 4

### Radioiodination of ODAM

**5-tributylstannyl-2-(2-dimethylaminomethylphenoxy)-benzyl alcohol (40)** A mixture of starting material 39 (30 mg, 0.08 mmol), bistributyltin (0.2 mL) and Pd(PPh₃)₄ (10 mg) in mixed solvent (2 mL, Et₃N:dioxane=1:1) was stirred at 90°C overnight. Solvent was removed and the residue was purified by PTLC (CH₂Cl₂:MeOH=9:1) to give 12 mg of product. ¹H NMR (CDCl₃, δ): 0.85-1.69 (m, 27H, SnBu₃), 2.25 (s. 6H, NCH₃), 3.50 (s, 2H, NCH₂), 4.35 (s, 2H. HOCH₂), 6.69 (d, 1H, J=8.1 Hz, ArH), 6.96 (d, 1H. J=7.8 Hz, ArH), 7.00 (d, 1H, J=8.4 Hz, ArH), 7.17 (d,d. 1H, J=7.7, 1.8 Hz, ArH). 7.22 (d,d, 1H, 3=7.2, 1.5 Hz, ArH),7.37 (s, 1H, ArH), 7.39 (d,d, 1H, J=7.5, 1.2 Hz, ArH), HRMS Calcd(C₂₈H₄₅NO₂Sn): M/Z 548.2550 (M⁺+1); Found M/Z 548.2548 (M⁺+1).

**5-[123-Iodo or 125-iodol-2-(2-dimethylaminomethylphenoxy)-benzyl** alcohol Radioiodination was carried out by iododestannylation. ODAM was reacted with radioactive sodium iodide (I-125 or I-123) in the presence of hydrogen peroxide, and purified by HPLC.

### Example 5

### Biological Activity of Compounds of the Invention

### General

Male Spague-Dawley rats weighing 225-275 g were used in this study. No carrier added [¹²⁵I]/[¹²³I] Nal (0.1N NaOH solution) were purchased from Nordion (Ottawa, Canada) and DuPont NEN Research Products (Boston. MA), respectively. [¹²⁵I]IPT was prepared according to the procedure described previously (Kung, M. P. *et al., Synapse 20(4):*316-324 (1995)). Paroxetine was generously provided by Di. C. Mathis of University of Pittsburgh. (+)McN5652 was kindly provided by Research Biochemicals Int. through a synthesis program supported by NIMH. Nisoxetine was prepared in our laboratory according to a method reported previously (Srebnik, M. *et al., J. Med. Chem.* 53:2916-2920 (1988)). Serotonin, methylphenidate and raclopride were purchased from Research Biochemicals Int. (Natick, MA). Desipramine and p-chloramphetamine (PCA) were purchased from Sigma Chemical Co. (St. Louis, MO). All other chemicals used were of reagent grade.

### Methods

### In Vitro Binding

### Cell Lines

Three monoamine transporters expressed respectively in a common parental cell line, LLC-PK, (referred to as LLC-DAT, LLC-NET and LLC-SERT) were kindly provided by Dr. G. Rudnick of Yale University. Cells were plated and grown to confluence as a monolayer as described (Gu, H. *et al., J. Biol. Chem. 269(10):*7124-7130 (1994)). The membrane homogenates were prepared as for for the binding assays with [¹²⁵I]IPT. Inhibition of compounds on [¹²⁵I]IPT binding to LLC-DAT, LLC-NET and LLC-SERT were carried out in the assay buffer (50mM Tris-HCl, pH 7.4,120 mM NaCl and 0.1 % BSA) containing 0.2-0.4 nM [¹²⁵I]IPT as described previously (Hou, C. *et al., Society for Neuroscience Program 28th Annual Meeting,* Los Angeles, CA [abstract 241.21]:112 (1998)). Under these conditions, IPT showed K, values of 1.2, 19.3 and 1.2 nM for DAT, NET and SERT, respectively.

### Native Tissues

The frontal cortex of rat brain were dissected and homogenized in 30 volumes of buffer (50 mM Tris-HCl, pH 7.4 containing 120 mM NaCl and 5 mM KCl). The homogenates were centrifuged at 20,000g for 20 min. The resulting pellets were then re-suspended and recentrifuged. The final pellets were suspended in the same buffer and used for *in vitro* binding assays.

Binding assays were performed in glass tubes (12 x 75 mm) with a final volume of 0.5 ml. In saturation experiments, aliquots (100 µl corresponding to 20-30 µg of protein) of membrane suspensions were mixed with buffer (as above) containing 0.05-2.0 nM [¹²⁵I]IDAM.

Competition experiments were performed using 0.2 nM [¹²⁵I]IDAM and 8-10 concentrations (10¹⁰- 10⁻⁵M) of competing drugs. Various inhibitors were serially diluted with the buffer containing 0.1% BSA to overcome the stickiness and loss due to dilution. Nonspecific binding was defined with 1 µM paroxetine. Incubation was carried out for 60 min at room temperature and then terminated by separation of bound from free radioligand by filtration through glass fiber filters (Schleicher & Schuell No. 25, Keene, NH) presoaked with 1% polyethylenimine. The filters were then washed three times with 3 ml of ice-cold 20 nM Tris buffer and counted in a gamma counter (Packard 5000) with 70% efficiency. To study the effect of N^{a+}, the homogenates were prepared in a sodium-free 50mM Tris-HCl/5 mM KCl buffer. Final Na⁺ concentrations of 0, 2, 5, 10, 20, 37.5, 75, 150 and 300 mM were utilized and the same assays were performed as described above. Protein determinations were performed with Lowry's method (Lowry, O. *H, et al., J. Biol. Chem. 193*:265-275 (1951)) using bovine serum albumin as a standard. The results of saturation and competition experiments were subjected to nonlinear regression analysis using EBDA (Munson, P. J. and D. *Rodbard, Anal. Biochem. 107(1)*:220-239 (1980)) by which to obtain K_{d}, Bₘₐₓ and IC₅₀ values.

### p-Chloroamphetamine (PCA) Lesion Studies

A neurotoxin, *p*-chloroamphetamine (PCA), was used to lesion the serotonergic nerve terminals of rats as described previously (Sanders-Bush, E. and V. J. Massari, *Fed. Proc. 36*:2149-2153 (1977)). Ten rats were divided into two groups, and they were injected daily with PCA (8 mg/kg, i.p.) or saline for 3 consecutive days, respectively. The animals were sacrificed 5 days after the last injection and the frontal cortical membrane homogenates were prepared. The difference in the binding sites (Bₘₐₓ) and affinities (K_{d}) for [¹²⁵I]IDAM were measured between control and PCA-lesioned rats.

### Statistics

Effects of drugs on the radioactivity in the various regions of CNS were examined using analysis of variance procedures. IDAM binding in drug-treated rats was compared against control animals in different brain regions. using one-tailed t-tests. The level of significance was defined as p<0.05, after Bonferroni correction for multiple comparisons.

### Biodistribution in Rats

Three or four rats per group were used for each biodistribution study. While under ether anesthesia, 0.2 ml of a saline solution containing 10 µCi of radioactive tracer was injected into the femoral vein. The rats were sacrificed at the time indicated by cardiac excision while under ether anesthesia. Organs of interest were removed and weighed, and the radioactivity was counted using a Packard automatic gamma counter (Model 5000). The percent dose per organ was calculated by a comparison of the tissue counts to counts of 1 % of the initial dose (100 times diluted aliquots of the injected material) measured at the same time.

Regional brain distribution in rats was measured after an i.v. injection of the radioactive tracer. Samples from different brain regions [cortex (CX), striatum (ST), hippocampus (HP), cerebellum (CB) and hypothalamus (HY)] were dissected, weighed and counted. The percentage dose/g of each sample was calculated by comparing sample counts with the counts of the diluted initial dose described above. The ratio of specific binding (SB) in each region was obtained by dividing the difference ofeach region from the cerebellum (percentage dose/g) with that of the cerebellum [(region-CB)/CB]. The cerebellum region containing no serotonin transporters was used as a background region.

*In vivo* competitive binding of various uptake of [¹²⁵I]IDAM was investigated by pretreating the animals respectively with paroxetine, (+)McN5652, desipramine, nisoxetine, methylphenidate. raclopride and IDAM (2 mg/kg, each, i.v. at 5 min prior to the injection of [¹²⁵I]IDAM). Experiments were performed using groups of 3-4 animals per study. Similar regional brain distributions were determined at 60 min after [¹²⁵I]IDAM injection as described above. The reduction of regional specific binding in the drug-pretreated rats was compared to the control animals with saline pretreatment.

### Metabolite Analysis of Brain Tissue

Sixty minutes after an i.v. injection of 200-300 µCi of [¹²⁵I]IDAM into two male rats, the areas corresponding to the hypothalamus region were dissected , individually. The tissues were then homogenized in 1.5 ml of 50 mM Tris buffer (pH 7.4) and the homogenates were extracted with ethyl acetate (3 x 1.5 ml) in the presence of the non-radioactive IDAM (100 µg). The extracted ethyl acetate layers were evaporated to near dryness and the purity of [¹²⁵I]IDAM in the condensed extracts was analyzed by HPLC (PRP-1 column; solvent: CH₃CN/DMGA: 90/10; flow rate: 1ml/min). Control samples were performed by adding 5-10 µCi of [¹²⁵I]IDAM to hypothalamus tissues followed by the same procedures used for the experimental samples to determine the extraction efficiency.

### Ex Vivo Autoradiography of[¹²⁵I]IDAM in Rat Brain

Rats under ether anesthesia were injected intravenously with 0.4 ml of a saline solution containing 500 µCi of[¹²⁵I]IDAM. At 60 min post i.v. injection, the animals were sacrificed by cardiac excision while under ether anesthesia. The brains were rapidly removed, placed in embedding medium (Tissue Tek, Miles Laboratory, Elkhart, IN) and frozen with powdered dry ice. After reaching equilibrium at -20°C consecutive 20 µm coronal sections were cut on a cryostat microtome (Hacker Instruments, Fairfield, NJ), thaw-mounted on microscope slides, and air-dried at room temperature. The slides containing the brain sections were exposed to DuPont x-ray film along with 20 µm thick ¹²⁵I standards (Amersham Corp., Arlington, III) for 72 hr in 9 autoradiographic cassetts. The microscale standards were calibrated with tissue mash standards for the value conversion to nCi/mg protein. The exposed film was developed by a Kodak automatic film processor. The optical densities were determined with an image analysis system developed by NIH (Image 1.61). The blocking studies were carried out by pretreating the rats with paroxetine or nisoxetine (2 mg/kg, i.v. 5 min prior to tracer injection). The brain sections of the pretreated rats were processed following the same procedures described above for the normal untreated rats.

### Results

### Selectivity for Monoamine Transporters (DAT, SERT and NET)

The binding selectivity for three monoamine transporters of IDAM, ODAM, other compounds of the invention and previously described compounds were compared. The results are reported in Tables 1A and 1B, below. These transporters, expressed respectively in a common parent cell line, LLC-PK₁, were used for the evaluation.

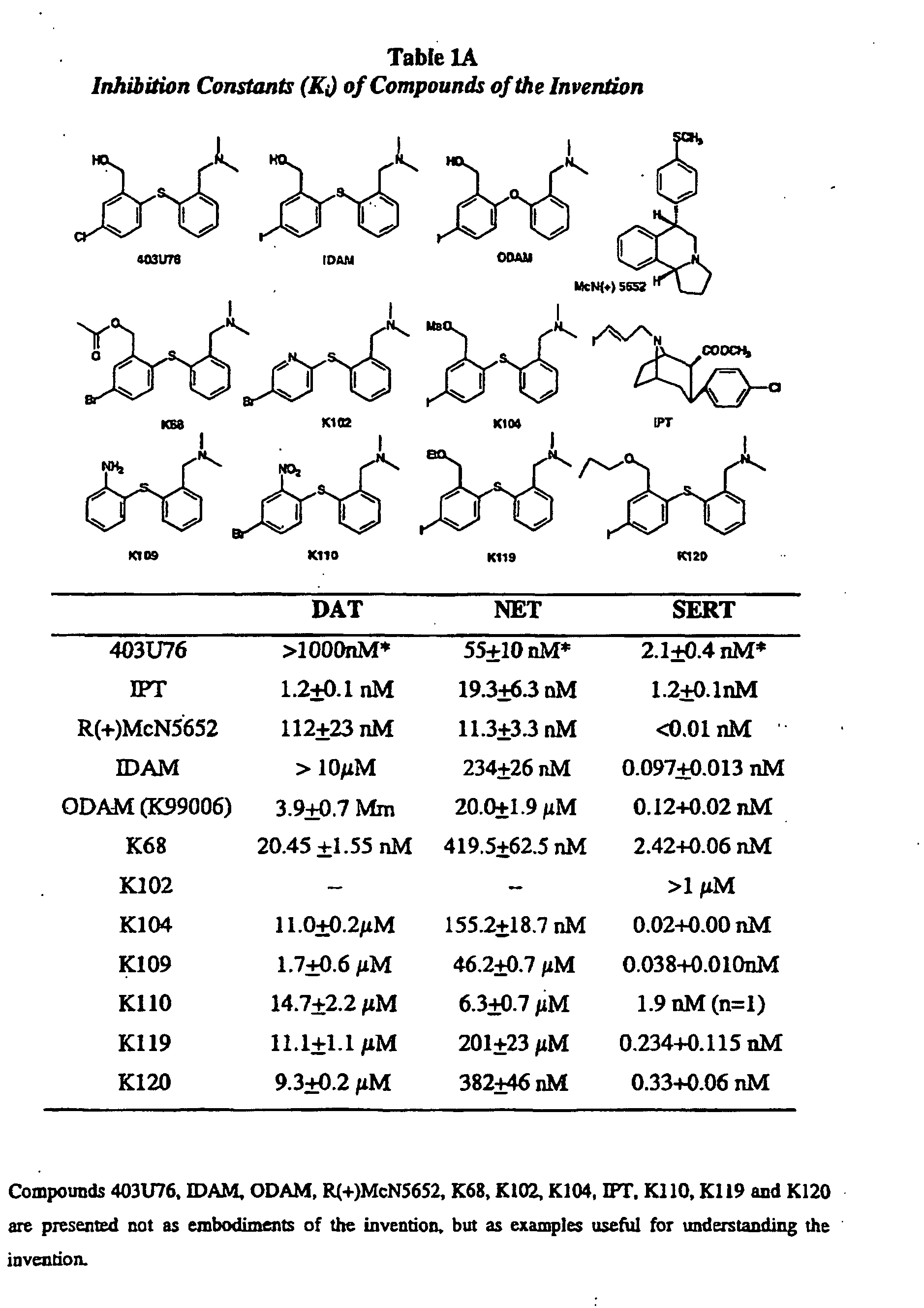

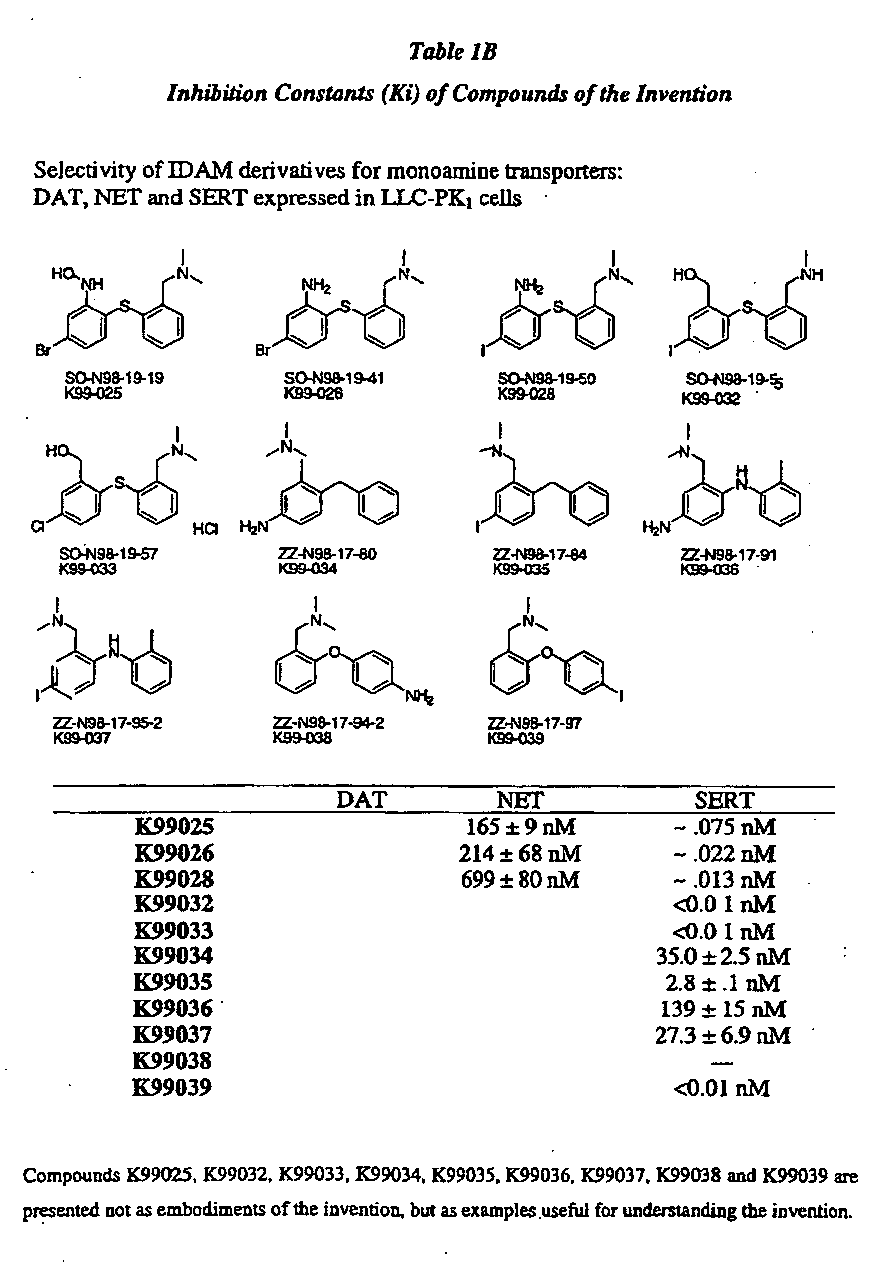

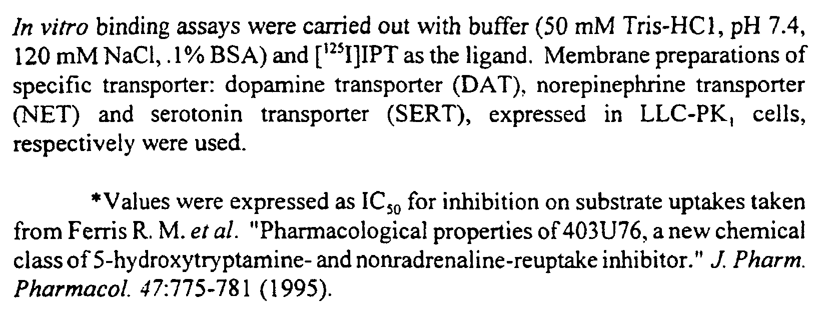

In a competition binding assay using [¹²⁵I]IPT as the radioligand for all three monoamine transporters (Kung, M. P. *et al., Synapse 20(4)*:316-324 (1995); Hou, C. *et al., Society for Neuroscience Program 28th Annual Meeting,* Los Angeles, CA [abstract 241.21]:112 (1998)), IDAM, as well as the brominated derivatives, displayed high affinities (Kᵢ values were less than 0.1 nM) for SERT. High Kᵢ values (> 10 µM) indicates the lack of binding of these compounds for DAT. The substitution of Br with I appeared to reduce the binding affinity to NET (K, = 35.4 nM for the brominated derivative and 234 nM for IDAM). These results suggested that IDAM is superior to the previously reported chlorinated derivative (403U76) (Ferris, R. M. *et al., J. Pharm. Pharmacol. 47*:775-781 (1995)) (selectivity shown in Table 1 was expressed as the inhibition on substrate uptakes). (+)McN5652, the selective PET tracer for SERT, was evaluated under similar assay conditions. A selective binding was observed for this compound with Kᵢ values of <0.01,11.3 and 112 nM for SERT, NET and DAT, respectively. Compared to (+)McN5652, IDAM displayed a comparable and even better selectivity profile (less prominent NET binding) for SERT.

### In Vitro [¹²⁵I]IDAM Binding Studies

Similar to other SERT ligands, [¹²⁵I]IDAM binding to rat frontal cortical membrane homogenates requires the presence of sodium ion (Na⁺). There was a rapid increase in specific binding with increasing sodium concentrations up to 100 mM; above that level there was a further gradual increase (about 10%) up to 200 mM concentration. Therefore, subsequent assays were performed using 50 mM Tris-HCl buffer containing 5 mM KCl and 120 mM NaCl.

Specific binding of [¹²⁵I]IDAM to SERT was examined using rat frontal cortical membrane homogenates. The non-specific binding was defined by using 1 µM paroxetine. Under these conditions, the specific binding was saturable and of high affinity. Scatchard transformation of the binding data gave a linear plot indicating one-site binding (FIG. 1). The mean values from 3 experiments gave a K_{d} value of 0.25 ± 0.05 nM and a Bₘₐₓ value of 272 ± 30 fmol/mg protein. The number of binding sites obtained with [¹²¹I]IDAM is comparable to the values reported with other similar SERT ligands (Kung, M. P. *et al., Life Sci. 51:95-106* (1992); Mathis, C. A. *et al., Eur. J. Pharmacol. 210(1)*:103-104 (1992)).

The systematic treatment of rats with *p*-chloroamphetamine (PCA) is known to reduce the endogenous serotonin level by 90% and cause a concomitant reduction of serotonin neurons in the brain (Kohler, C. *et al., Ann. NYAcad. Sci. 305*:645-663 (1978)). The frontal cortex tissue preparation of rats treated with PCA was used for binding studies of [¹²⁵I]IDAM. Lesions of the serotonergic neuron were induced by systemic injection of PCA, which produced a profound effect on binding to the cortical membrane homogenates prepared from the lesioned animals (FIG. 1). As expected, there was a dramatic reduction of specific binding: the observed Bₘₐₓ decreased from 272 ± 30 fmol/mg protein to 20 ± 7 fmol/mg protein with no significant change on the binding affinity (K_{d}).

### In Vitro Competition Studies

A number of drugs were used in competition binding assays with [¹²⁵I]IDAM to characterize the pharmacological profiles of [¹²⁵I]IDAM in rat frontal cortical homogenate. Paroxetine and (+)McN5652, two known selective ligands for SERT, compete effectively with [¹²⁵I]IDAM binding showing Kᵢ values less than 0.1 nM (FIG. 2). The non-radioactive IDAM also showed a high potency in inhibition with a low Kᵢ value (Kᵢ = 0.08 nM). Drugs such as nisoxetine and desipramine, known selective MET inhibitors, displayed a moderate affinity to compete for [¹²⁵I]IDAM binding (Kᵢ = 20.2 and 54.9 nM for nisoxetine and desipramine respectively). Whereas 5-HT, the endogenous neurotransmitter, gave a Kᵢ value of 437 nM. Raclopride (a selective dopamine D2/D3 ligand) and methylphenidate (a selective DAT ligand) were much less active in inhibiting [¹²⁵I]IDAM binding (Kᵢ, = 669 and>1000 nM, respectively).

### In Vivo [¹²⁵I]IDAM Binding

Biodistribution of [¹²⁵I]IDAM in different organs and the brain regions after i.v. injection is shown in Table 2 below. Initial brain uptake at 2 min. post-injection was high (2.44 % dose), indicating an efficient passage of the tracer through the intact blood-brain barrier. The optimal lipophilicity of this ligand was reflected by its partition coefficient (PC = 473) between n-octanol and phosphate buffer, pH 7.4. The total radioactivity recovered at 2 min post-injection from the major organs was relatively low (less than 60% of total injected dose). The fast clearance of [¹²⁵I]IDAM from the rats suggests that the compound may be rapidly metabolized and excreted into urine. At the later time points, the radioactivity was washed out from the brain with 0.50 and 0.17% dose at 60 and 120 min, respectively. The majority of the radioactivity was washed out from the brain by 4 hr after injection.

Radioactivity levels in all brain regions were the highest at 2 min and then decreased over the 4-hr period. The washout rate was faster in CB, where no SERT binding sites are located, as compared to other brain regions (i.e. ST, HP, CX and HY where different levels of SERT are concentrated). At 30 min post-injection, regions containing serotonergic innervation, that is, ST, HP, CX and HY, showed higher concentrations of radioactivity than CB. The ratio of specific uptake in hypothalamus region ([HY-CB/ CB) increased consistently up to 2 hr after injection (Table 2). However, the regional activities appeared to fall rapidly between 60 min and 120 min post-injection. Based on this observation the drug-challenge experiments were performed at 60 min post i.v. injection.

To investigate the stability of [¹²⁵I]IDAM in rat brain after an i.v. injection, radioactivity associated with hypothalamus region at 60 min postinjection was examined. It was found that only unmetabolized [¹²⁵I]IDAM (> 95%) was recovered. No significant detectable metabolites in the brain re-enforces the favorable characteristic of the tracer.

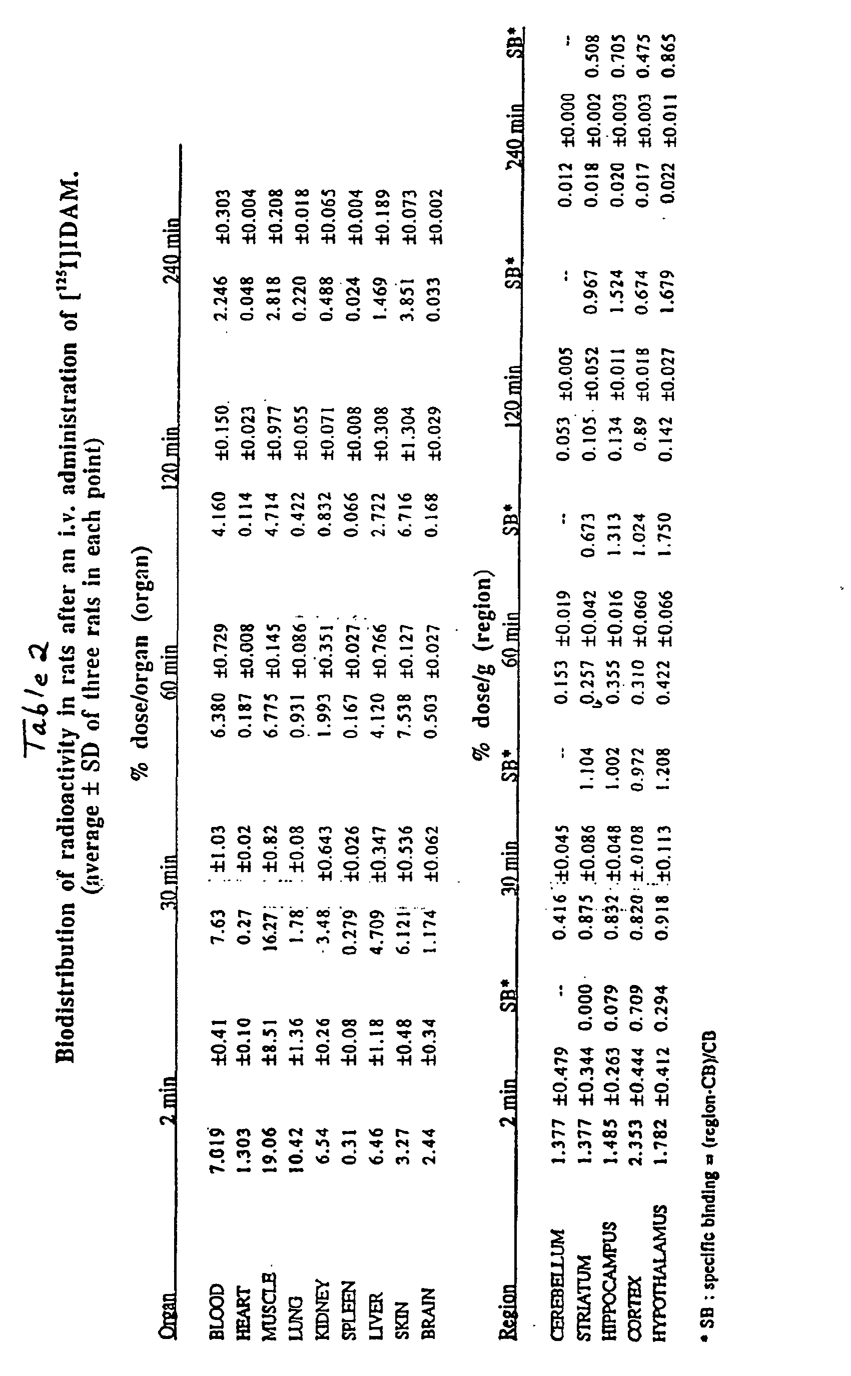

### Pharmacological Specificity of In Vivo [¹²⁵I]IDAM Binding

Effects of pretreatment of rats with various compounds on regional brain distribution of [¹²⁵I]IDAM were examined to assess the *in vivo* pharmacological specificity. Specific binding of [¹²⁵I]IDAM in all brain regions, except HP, was dramatically reduced by pretreatment with paroxetine, (+)McN5652 and IDAM (p<0.05). These results suggest a *potent in vivo* competition binding of these compounds with [¹²¹I]IDAM for SERT. The magnitude of blocking in different regions was: HY>CX>ST>HP (FIG. 3). There was a high level of non-specific binding, which could not be blocked by specific SERT ligands, in HP region. Compounds such as methylphenidate (selective for DAT) and raclopride (selective for dopamine D2/D3 receptors) showed no effect on specific. uptake. Nisoxetine and desipramine, two selective ligands for NET, showed no significant inhibition on [¹²⁵I]IDAM binding to the brain region, indicating the lack of binding to this tracer to NET sites. Highly selective *in vivo* binding of SERT observed with [¹²⁵I]IDAM is a very desirable property for a potential SERT imaging agent.

### Ex Vivo Autoradiography

At 60 min post-injection of [¹²⁵I]IDAM. autoradiograms of rat brain sections showed intense labeling in several regions (Paxiriog, G. and C. Watson *"The Rat Brain In Stereotaxic Coordinates,"* New York Academic Press, (1986)), i.e. olfactory tubercle, lateral septal nucleus, hypothalamic and thalamic nuclei, globus pallidus, central gray, superior colliculus, substantia nigra, interpeduncular nucleus, dorsal and median raphes and locus, coerulus (FIG. 4), areas known to have high densities of SERT sites (Cortes, R*. et al., Neuroscience 27*:473-496 (1988)). Lower but detectable labeling was also found in frontal cortex, caudate putamen, ventral pallidum and hippocampus, areas containing a significantly lower amount of SERT sites. The regional distribution observed with [¹²⁵I]IDAM is consistent with those reported for other SERT ligand (Biegon, A. *et al., Brain Res. 619*:236-246 (1993); Kovachich, G. B. *et al., Brain Res. 454*:78-88 (1988); De Souza, E. B. and B. L. Kuyatt, *Synapse 1*:488-496 (1987)) indicating that *in vivo* nonspecific binding of [¹²⁵I]IDAM is insignificant. In rats pretreated with a high dose of paroxetine (2 mg/kg, i.v.), a selective ligand for SERT, the autoradiograms were significantly reduced as compared to the matched sections of the control rats. The percentage of specific binding varied from more than 95% in interperduncular nucleus, median raphe and dorsomedial hypothalamus to less than 30% in hippocampal regions and several cortical regions. High nonspecific binding (non-paroxetine blockable) observed in HP (more than 70%) observed by *ex vivo* autoradiogram correlated well with the results obtained using the dissected brain regions (FIG.3). Autoradiograms of brain sections from nisoxetine-pretreated rats indicated there is no statistically significant difference for labeling of [¹²⁵I]IDAM between control and nisoxetine-pretreated rats. The results further confirm the selective binding of[¹²⁵I]IDAM to SERT sites without concomitant labeling for NET sites.

### Example 5

### Biological Activity of ODAM (Phenoxetine)

Initial biodistribution study in rats (i. v. injection) as performed in Example 4 showed a rapid brain uptake and wash-out (2.03, 1.49, 0.79, 0.27 and 0.07% dose/organ at 2, 30, 60, 120 and 240 min, respectively). More importantly the hypothalamus region where the serotonin neurons are located exhibited a high specific uptake (Table 3). Hypothalamus/cerebellum ratios based on a percentage dose per gram (% dose/g) of these two regions showed values of 1.22, 1.86, 1.86, 1.63 and 1.34 at 2, 30, 60, 120 and 240 min, post *i.v*. injection, respectively. However, regional brain localization is not a clear cut as those of IDAM.

**Table 3**

| **Biodistribution in rats after an i.v. injection of [**^{**125**}**I]ODAM (% dose/organ, avg of 3 rats ±SD)** | | | | | | |
|---|---|---|---|---|---|---|
| Organ | 2 min | 30 min | 60 min | 120 min | | 240 min |
| BLOOD | 3.009 ±0.212 | 3.517± 0.504 | 3.296 ±0.373 | 1.868±0.186 | | 0.976 ±0.073 |
| BRAIN | 2.033 ±0.087 | 1.489±0.146 | 0.785 ±0.23) | 0.267 ±0.066 | | 0.065 ±0.017 |
| THYROl D | 0.080 ±0.019 | 0.039 ±0.006 | 0.028 ±0.005 | 0.034 ±0.006 | | 0.041 ±0.038 |

| **Regional brain distribution (ratio/CB)** | | | | | | |
|---|---|---|---|---|---|---|
| Region | | 2 min | 30 min | 60 min | 120 min | 240 min |
| CEREBELLUM | | 1.000 | 1.000 | 1.000 | 1.000 | 1.000 |
| STRIATUM | | 0.906 | 1.590 | 1.605 | 1.617 | 1.539 |
| HIPPOCAMPUS | | 0.983 | 1.563 | 1.791 | 1.846 | 1.648 |
| CORTEX | | 1.436 | 1.771 | 1.690 | 1.672 | 1.322 |
| HYPOTHALAMUS | | 1.221 | 1.860 | 1.858 | 1.630 | 1.343 |

| | | | | | | |
|---|---|---|---|---|---|---|
| PC:305; 1-octanol/0.1M KH₂PO₄ buffer, pH 7.4 | | | | | | |

Initial binding study using frontal cortex membrane homogenates of rat brain (see Example 4) and [¹²⁵I]IDAM as the ligand showed that the ODAM displayed a very good binding affinity for SERT (Kᵢ = 2.88±0.88 nM).

Using the membrane preparations containing specific monoamine transporter: DAT. NET and SERT expressed in LLC-PK, cells. respectively, the inhibition constant of IDAM and ODAM were determined and are listed in Table 4. It appears that IDAM is more potent and selective to SERT. However, the *in vivo* biodistribution data in rats and the SPECT imaging study suggest that ODAM displayed a higher brain uptake and slower clearance from the target area.

Table 4 is a comparison of inhibition constants (Kᵢ) of IDAM and ODAM using *in vitro* binding assays and membrane preparations containing specific monoamine transporters: DAT, NET and SERT expressed in LLC-PK, cells, respectively.

**Table 4 Comparison of Inhibition Constants (Kᵢ) of IDAM and ODAM**

| | DAT | NET | SERT |
|---|---|---|---|
| IDAM | > 10 µM | 234±26nM | 0.097 ± 0.013 nM |
| ODAM | 3.9 ± 0.7 µM | 20.0 ±1.9 nM | 0.12 ± 0.02 nM |

### Example 6

### SPECT Imaging in Baboons

The acquisition of SPECT images were obtained by a triple-head gamma camera equipped with ultra high resolution fan-beam collimators (Picker Prism 3000). The acquisition parameters comprised of a rotational radius of 14 cm, a 15% energy window centered on 159 KeV, 120 projection angles over 360 degrees, and a 128, x 128 matrix with a pixel width of 2.11 mm in the projection domain. Data collection started at immediately after iv. injection of 555 MBq ( 15 mCi) of [¹²³I]IDAM. Forty-eight 5-minute scans were carried out over a total time period of 240 min. The projection images were reconstructed by filtered-back projection. Then. a 3D, low pass Butterworth filter was applied. For uniform attenuation correction, Chang's first order method was used. A summed data between 60-120 min after injection of [¹²³I]IDAM were used to provide transverse, coronal and saggital views of SPECT images of the baboon head.

The SPECT imaging study of [¹²⁵I]IDAM in the brain of a baboon by SPECT, at 60-120 min after injection, showed an excellent contrast in the midbrain area (raphe nucleus, substantia nigra, hypothalamus), where a high density of SERT is concentrated (FIG. 5). Coregistration with MRI was used for the identification of the anatomical structure and resulted in the expected localization of this tracer. The image observed with [¹²⁵I]IDAM showed a very good correlation with the picture obtained with the PET imaging agent [¹¹C](+)McN5652.

SPECT images of [¹²³I]ODAM binding to serotonin transporters in a baboon's brain and the time activity curves were obtained in a similar manner (FIG 8A). The SPECT images obtained approximately at the level of the midbrain at 90-120 min post-injection were very similar to those obtained with [¹²³I]IDAM.

Time-activity curves for the midbrain and cerebellum for both [¹²³I]IDAM and [¹²³I]ODAM (both in the same baboon) were compared (FIG. 8B). The counts have been corrected for injected dose, so they should represent approximately the amount of relative brain uptake between the two tracers. [¹²³I]ODAM appears to have a higher brain uptake, and slower kinetics than [¹²³I]IDAM, possibly reflecting the slower metabolism expected for the newer tracer. Compared with [¹²³I]IDAM, the total integrated brain uptake (area under the time-activity curves extrapolated to *t* = ∞) is 46% higher in the cerebellum and 58% higher in the midbrain for [^{I23}I]ODAM. The kinetics of uptake and retention of ODAM in the brain displays different rates as compared with those of [¹²³I]IDAM.

FIG. 8A depicts a summed transverse image at the point of pseudo-equilibrium (approx. 90-120 min post-injection) approximately at the level of the midbrain.

FIG. 8B depicts time-activity curves for the midbrain and cerebellum for both [¹²³I]IDAM and [¹²³I]ODAM (both in the same baboon).

### Discussion of In Vitro and In Vivo Results

A novel radioiodinated compound, [¹²⁵I]IDAM, for mapping SERT in the brain was successfully evaluated. Initial studies on the binding selectivity of IDAM for three monoamine transporters (SERT. DAT and NET) were performed in LLC-PK₁ cells (expressing one of the three monoamine transporters, respectively) (Gu, H. *et al., J. Biol. Chem. 269(10)*:7124-7130 (1994)). IDAM displayed an excellent binding to SERT sites (Kᵢ = 0.097 nM) and showed a better selectivity for SERT (more than 1,000 fold), as compared to the corresponding chlorinated and brominated derivatives (Table 1). The lower affinity of IDAM for NET (Kᵢ = 234 nM) makes it highly desirable as a selective SPECT imaging agent for SERT. The selectivity for SERT demonstrated by IDAM is is similar or even better than that of (+)McN5652. This unique characteristic plus an optimal lipophilicity displayed by this tracer (partition coefficient = 473; 1-octanol/pH 7 buffer) makes this tracer a suitable candidate for a SERT tracer for SPECT imaging.

The binding affinity of this ligand was measured in a native tissue homogenate system, by using rat frontal cortical membranes, to which a high binding affinity of [¹²⁵I]IDAM (K_{d} = 0.2 nM) was observed. In addition, [¹²⁵I]IDAM displayed a lower nonspecific binding (<5% at K_{d} value) as compared to another radioiodinated ligand. 4-I-tomoxetine, (>25% at value: K_{d} = 0.04 nM) (Kung, M. P. *et al., Life Sci. 51:*95-106 (1992)). Several lines of evidence suggest that the specific [¹²⁵I]IDAM binding to rat cortical membranes is indeed associated with SERT. First, [¹²⁵I]IDAM binding to rat cortical membranes was inhibited effectively and completely by known SERT ligands, such as paroxetine and (+)McN5652 with Kₜ values in low nanomolar range. Drugs labeling the other two monoamine transporters, such as nisoxetine or desipramiue (NET selective) and methylphenidate (DAT selective) were much less potent in inhibiting [¹²⁵I]IDAM binding to the cortical membrane preparation (FIG. 2).. Second, destruction of serotonergic neurons by PCA resulted in a 90% loss of [¹²⁵I]IDAM binding to rat cortical membranes (FIG. 1), showing the ligand is specifically bound to the serotonergic neuron, on which the SERT binding sites are located.

In the *in vivo* biodistribution studies, a high accumulation of radioactivity in the rat-brain after an i.v. administration of [¹²¹I]IDAM was observed (2.44% of injected dose at 2 min post-injection), indicating its excellent ability to penetrate the blood-brain barrier. This is most likely due to the optimal lipophilicity displayed by this tracer (partition coefficient = 473). A significant amount of the injected [¹²⁵I]IDAM can be delivered to the target sites in the brain, which fulfills the first requirement as an useful imaging agent. At 60 min post-injection of [¹²⁵I]IDAM the regional distribution of radioactivity in the brain correlated well with the serotonergic innervation throughout the rat brain. Since the cerebellum region receives a minimal serotonin innervation (Hrdina, P. D. *et al., J Pharmacol. Exp. Ther. 252(1)*:410-418 (1990)), low radioactivity associated with cerebellum reflects the low *in vivo* nonspecific binding of [¹²⁵I]IDAM. The values of specific regional binding (SB) expressed as ratios [(region-CB)/CB] were excellent (see Table 2). These ratios obtained with [¹²⁵I]IDAM in hypothalamus region appear to be slightly lower than those reported for [¹¹C](+)McN5652 (3.6 at 90 min) or [¹²³I)5-I-6-NO₂-quipazine (4.0 at 2 to 6 hr) (Biegon, A. *et al., Brain Res. 619*:236-246 (1993)). The lower ratio (1.75 at 60 min) could be due to the limitation on the precision of dissecting small and discrete areas containing enriched SERT in the rat brain. The ratios reported from different laboratories are highly operator-dependent. Nevertheless, it is demonstrated that the specific localization of [¹²⁵I]IDAM in brain regions correlated well with the serotonergenic terminal distribution. The nature of specific regional binding of [¹²⁵I]IDAM, predominately in hypothalamus region, is further validated by competition studies. Virtually all of the selective binding (SB), as indicated by (HY-CB)/CB ratio, is blocked by a pretreatment with specific SERT ligands, i.e. paroxetine or (+)McN5652, (FIG. 3) suggesting [^{I25}I]IDAM is competing with these blockers for the same SERT sites. As expected, pretreatment with agents showing no SERT affinity did not affect the regional distribution of [¹²⁵I]IDAM in the rat brain. The *in vivo* pharmacological specificity of[^{I25}I]IDAM for SERT in the rat brain is well depicted in the present study.

The kinetics of *in vivo* [¹²⁵I]IDAM binding to SERT sites in the rat brain was found to be relatively fast, approaching the optimal ratio (target vs. nontarget) as early as 30 min post-injection. A rapid clearance from the brain regions was observed with a half-time of less than 60 min. In the present study with rats, a moderate peak ratio [SB = 1.75 of (HY-CB)/CB] was observed. Despite the fast peripheral metabolism, clearance and the moderate target vs. nontarget ratio observed with [¹²³I]IDAM in rats, the preliminary imaging studies with [¹²³I]IDAM/SPECT in baboons resulted in an excellent contrast and clearly reflected the known pattern of distribution of SERT in the brain of baboons (FIG. 5).

In conclusion, radioiodinated IDAM is a *selective in vivo and in vitro* ligand for SERT. This novel tracer, which demonstrated a high affinity, excellent selectivity and good brain penetration, has excellent characteristics for SPECT imaging of SERT in the brain.

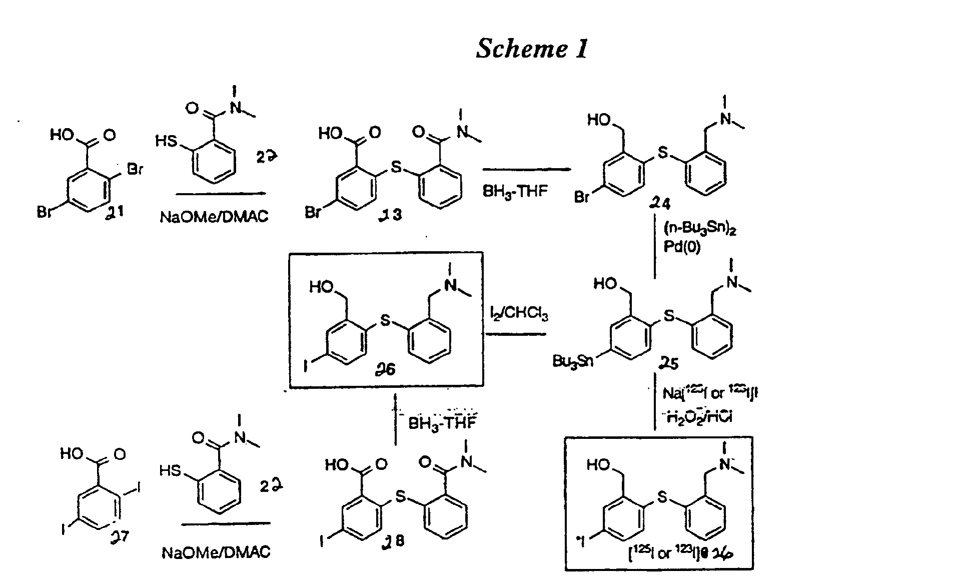

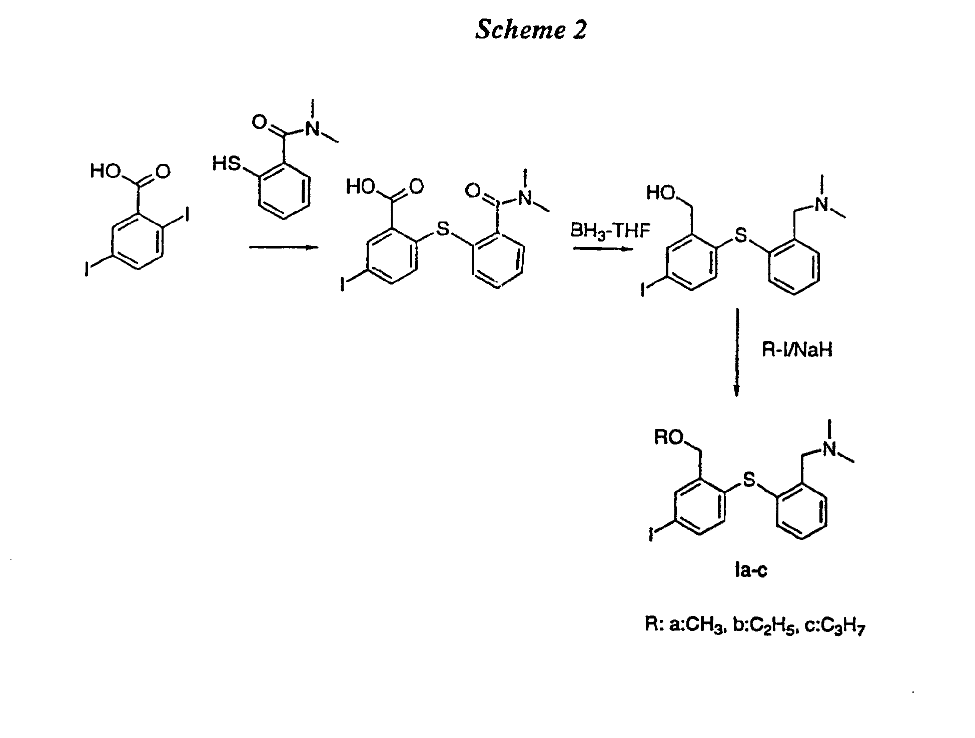

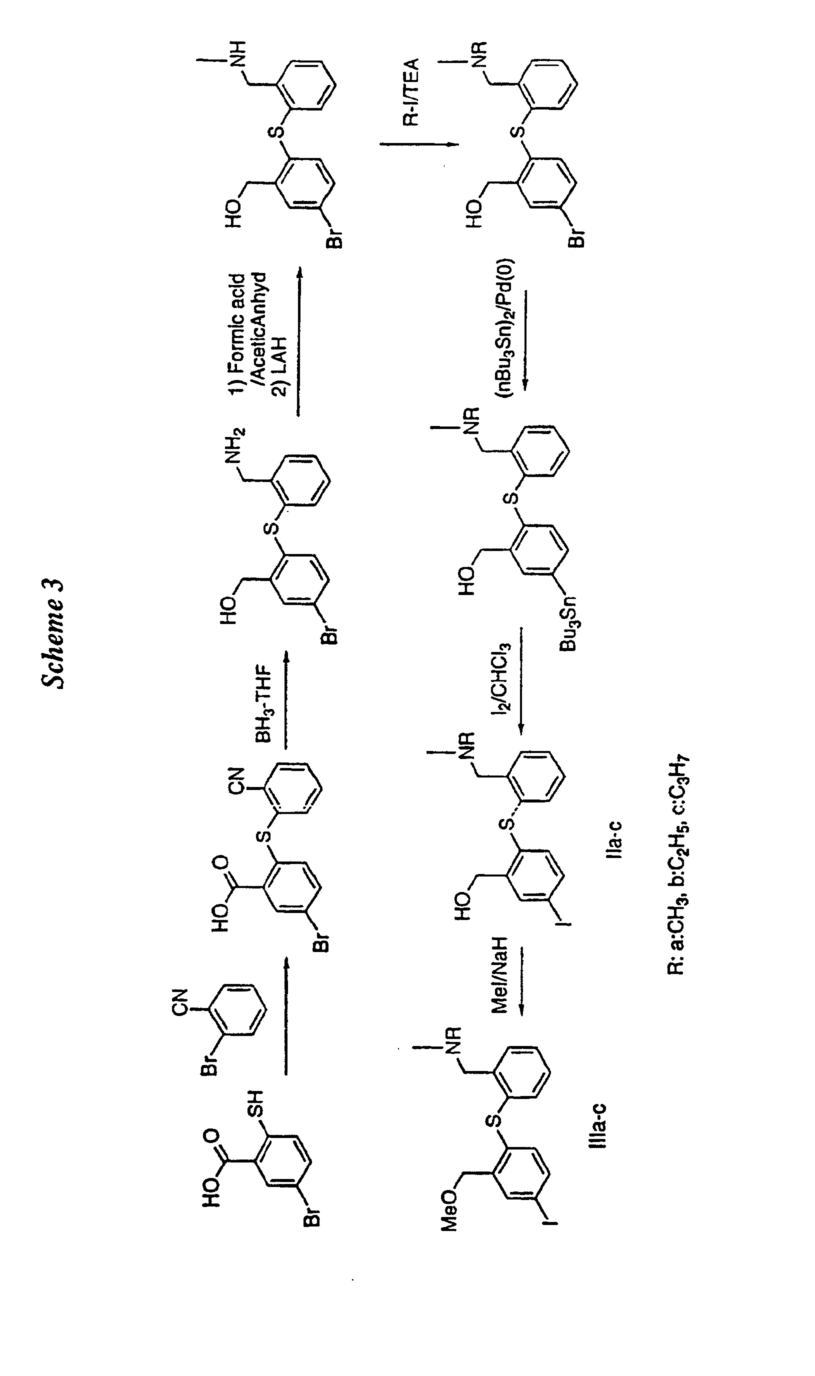

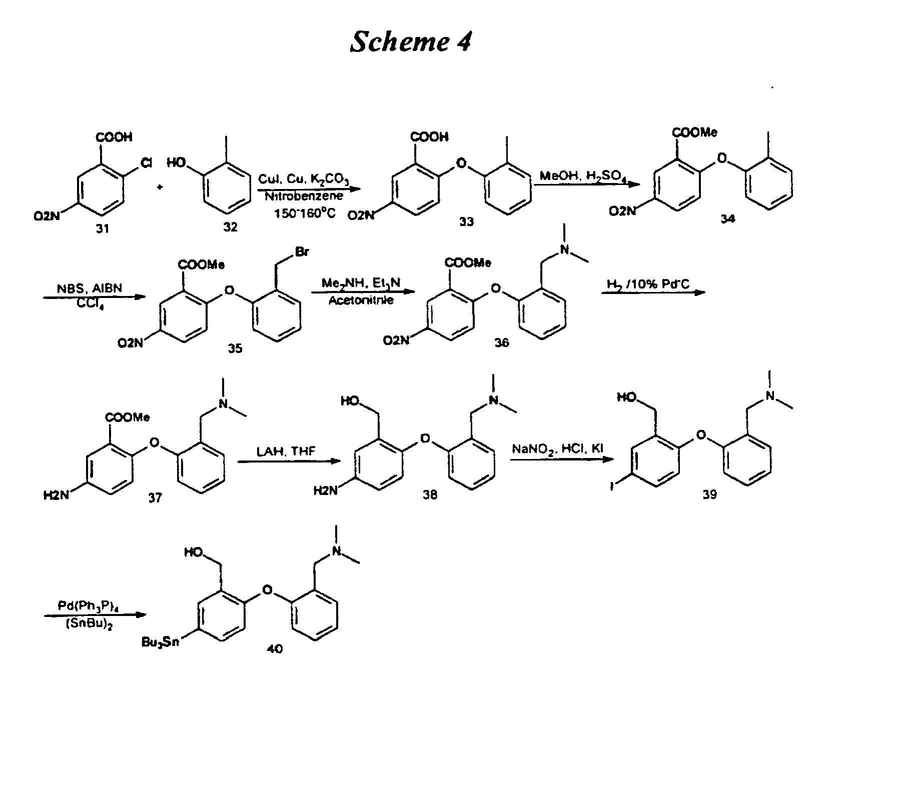

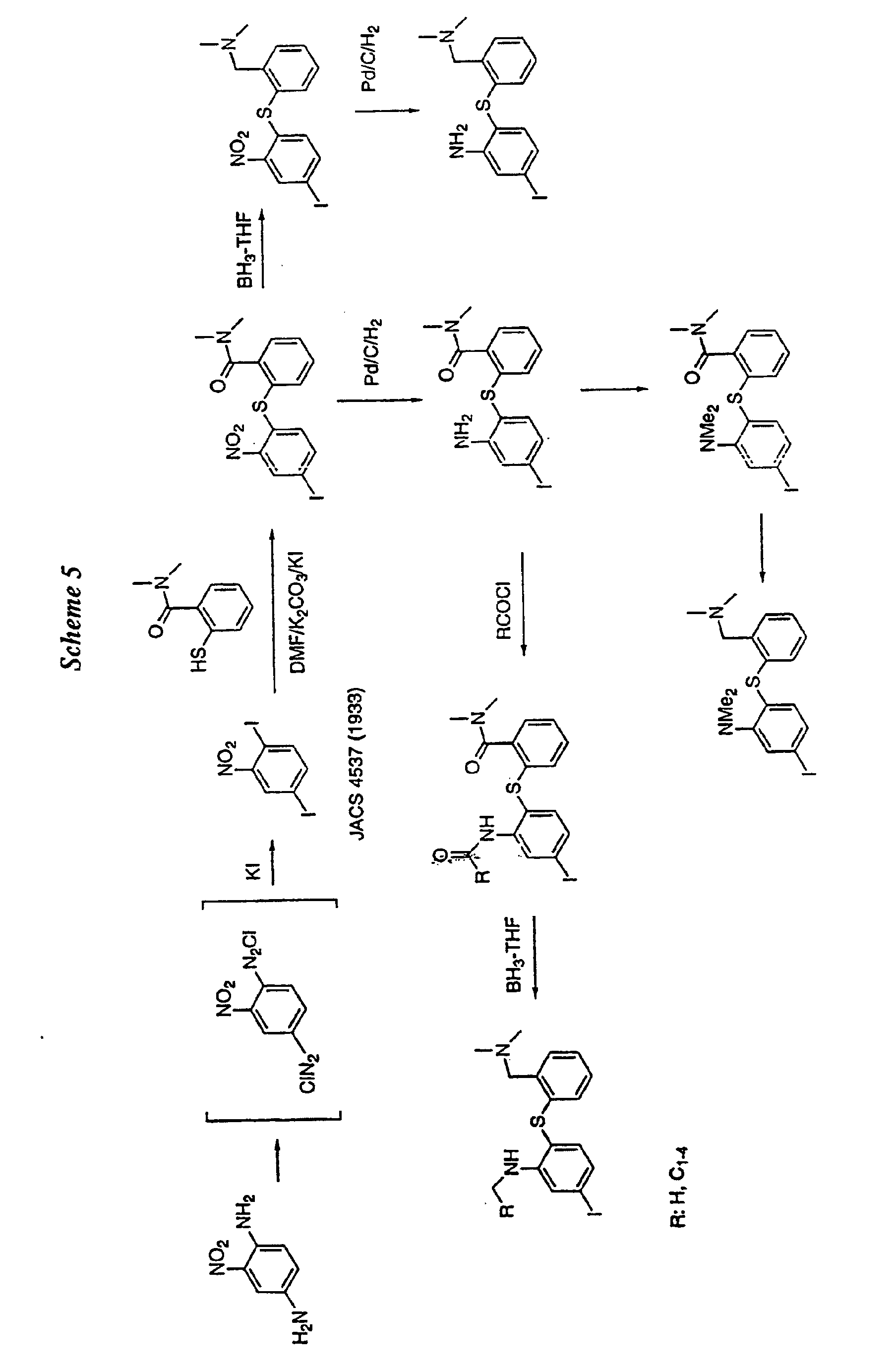

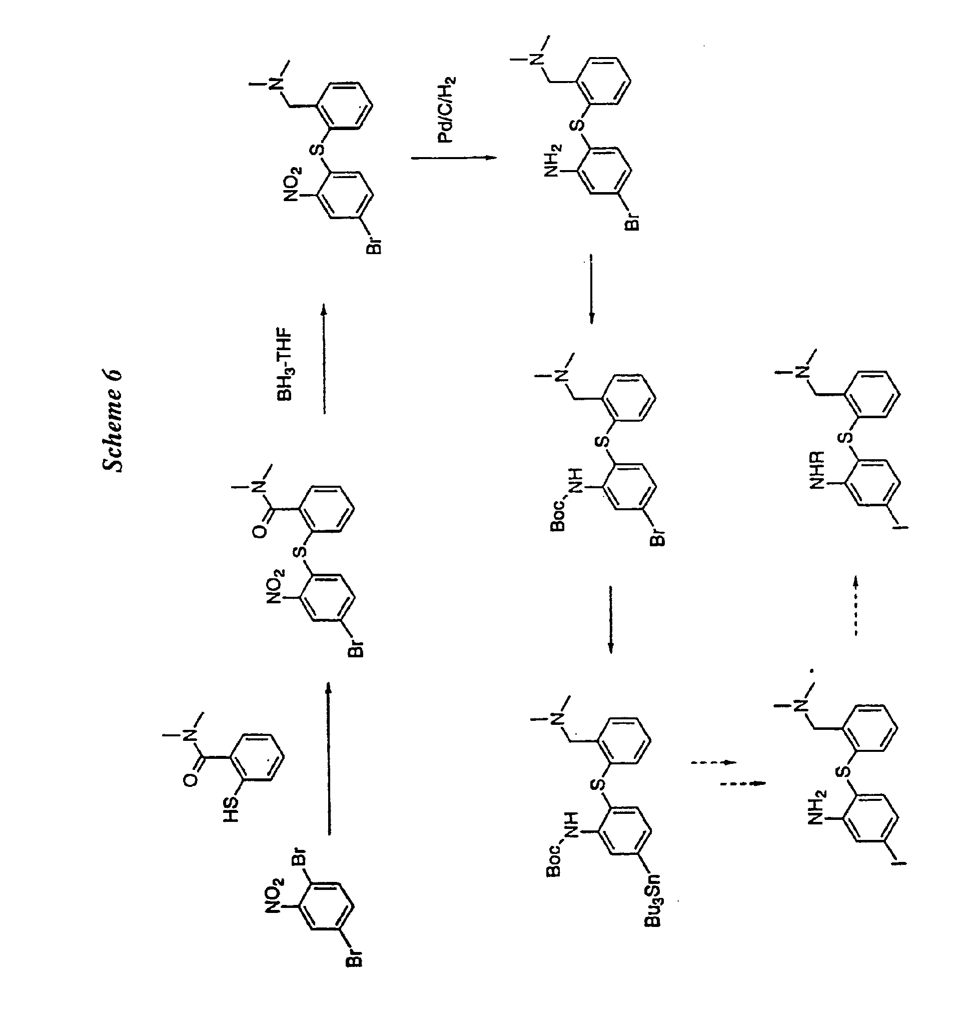

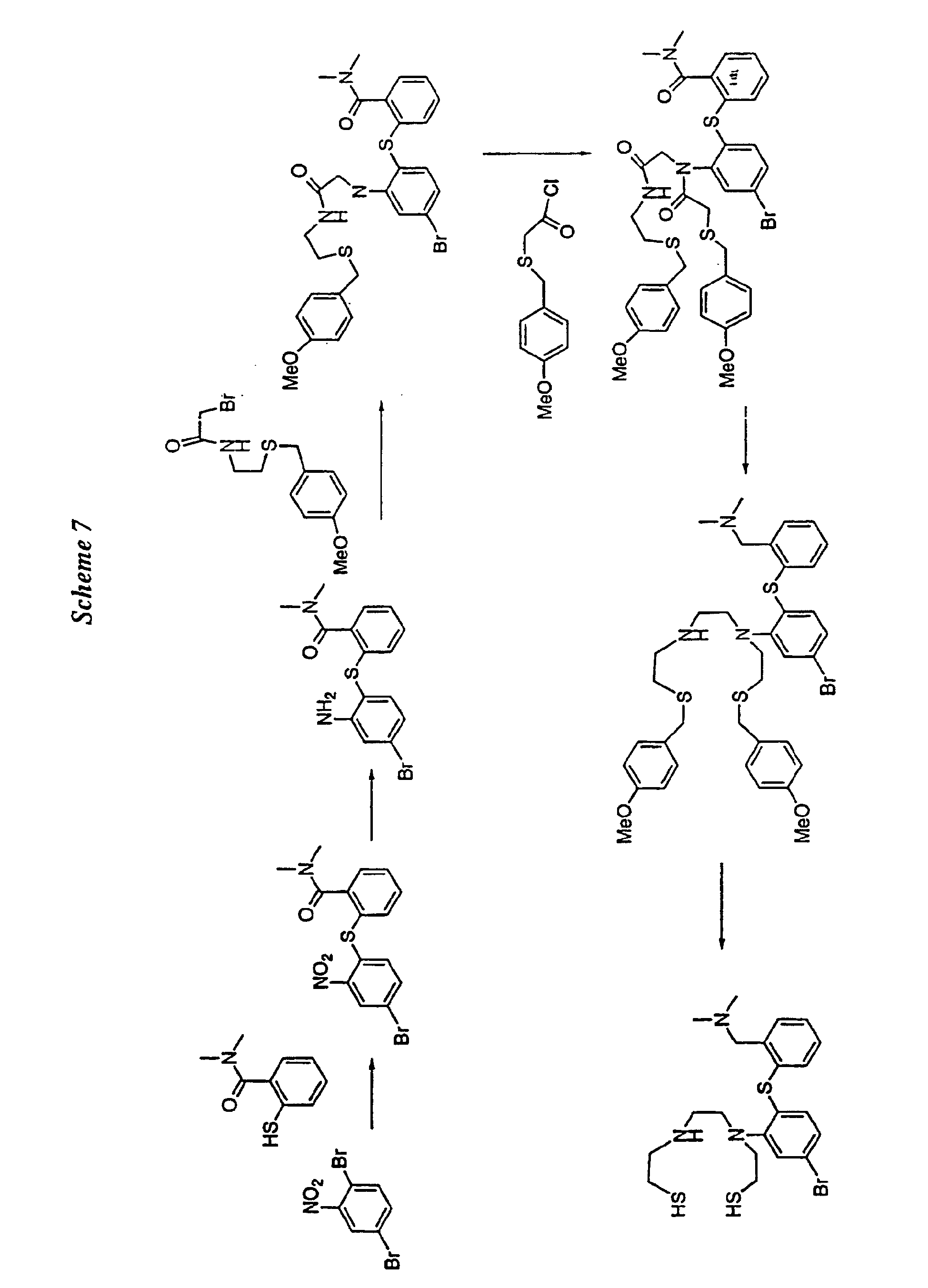

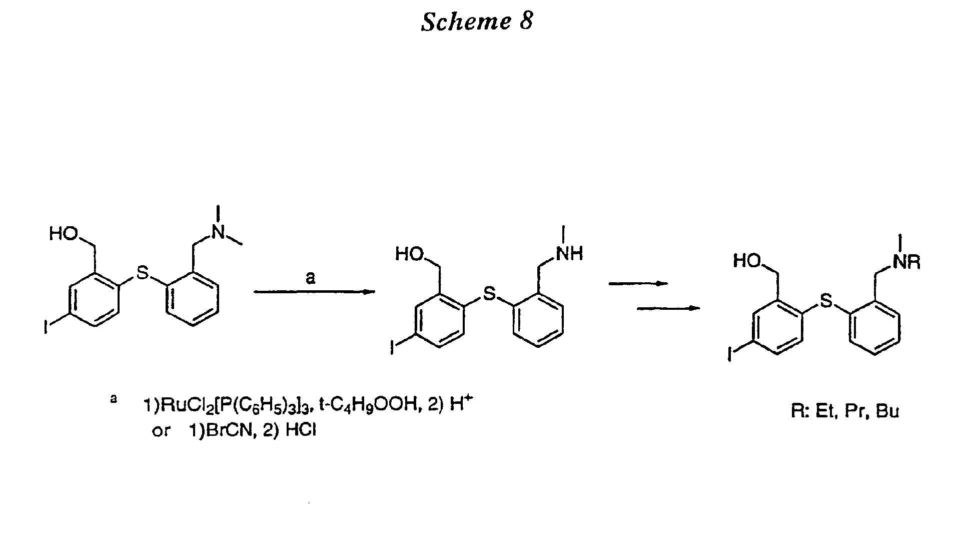

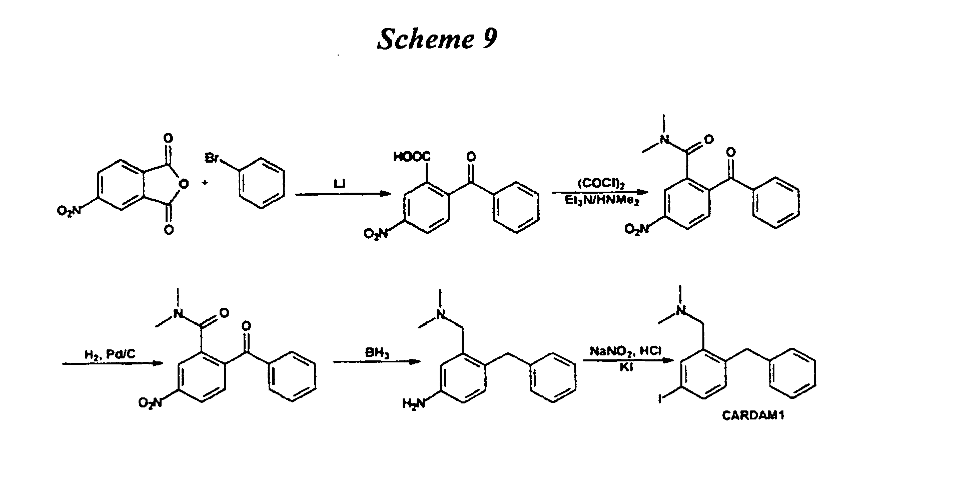

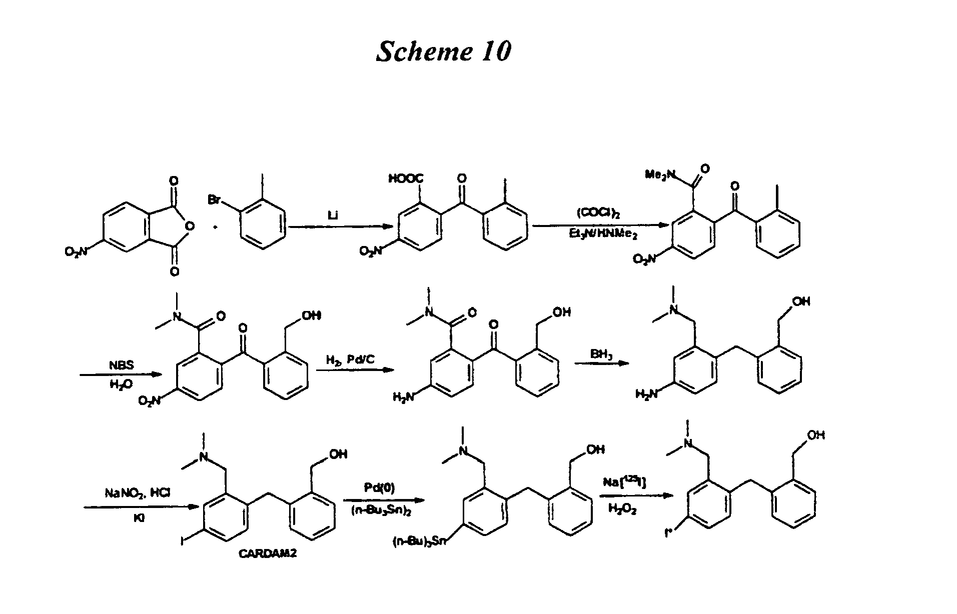

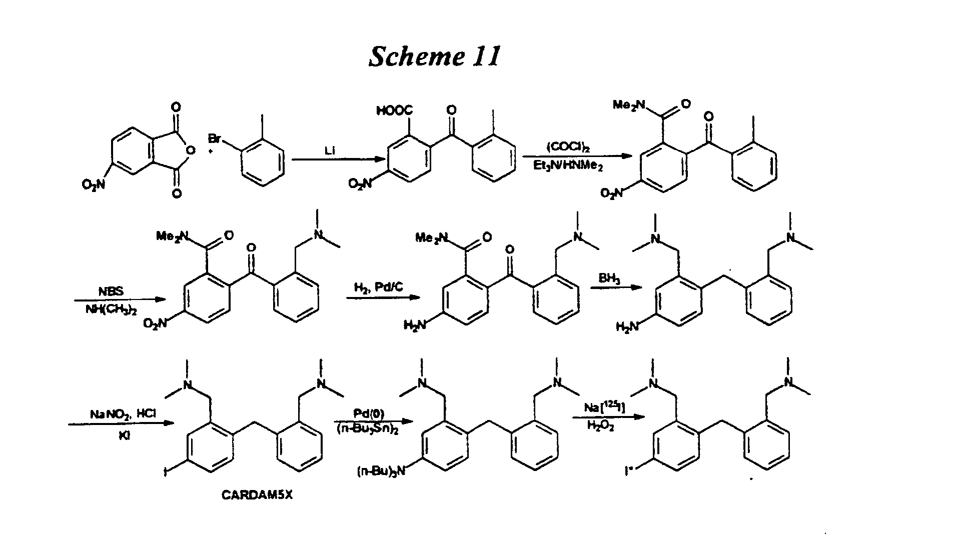

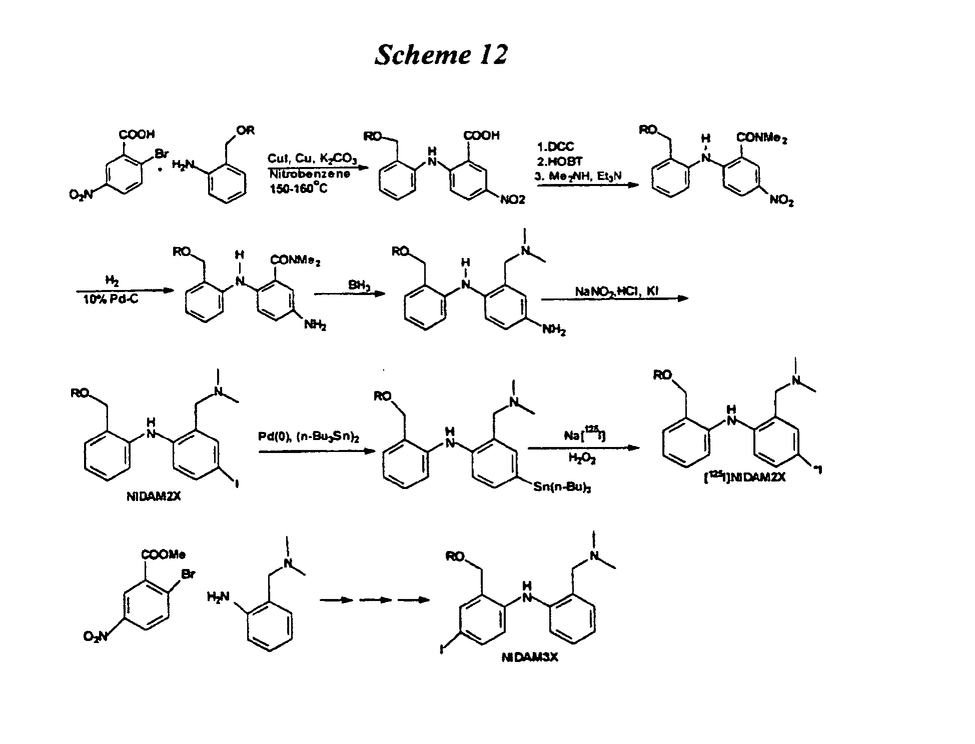

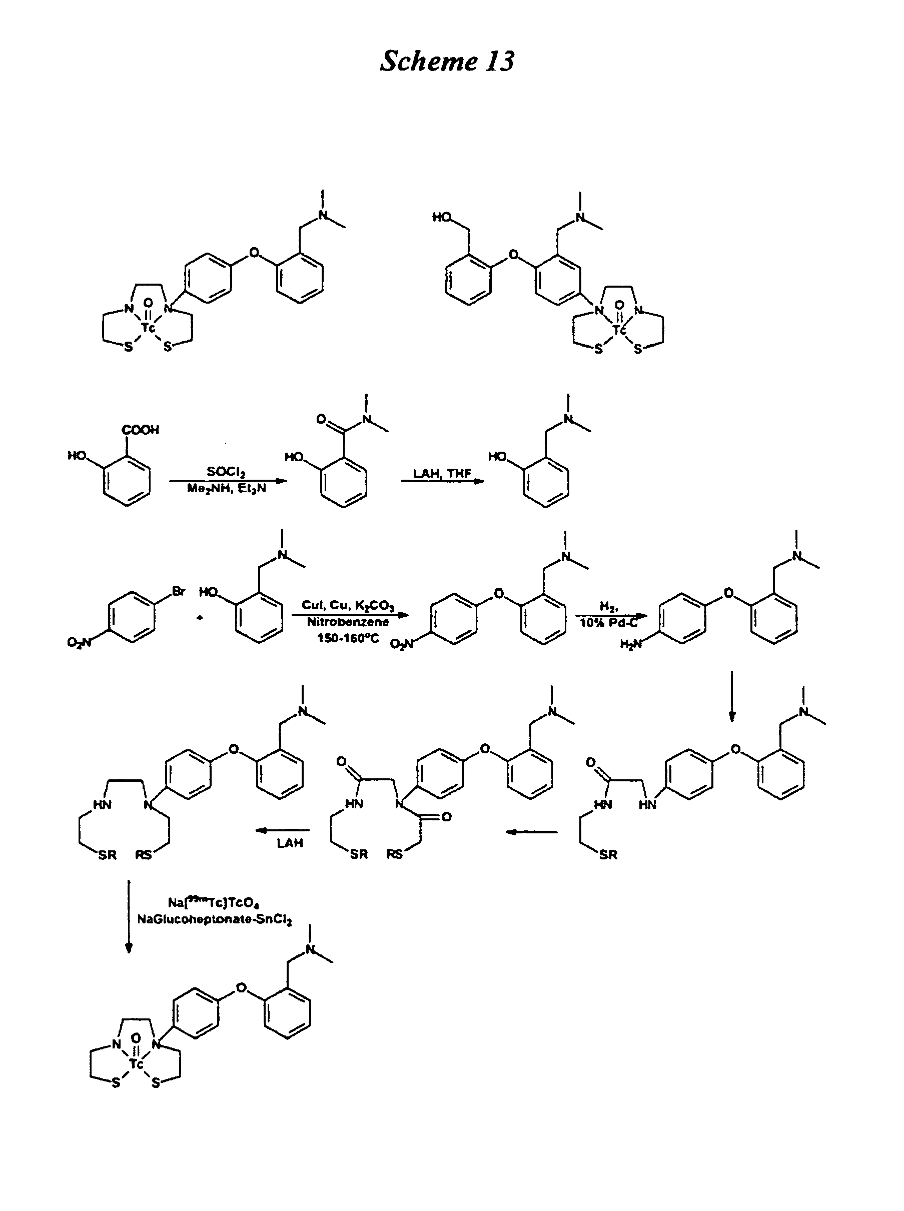

## Claims

1. A compound of Formula ***I***: 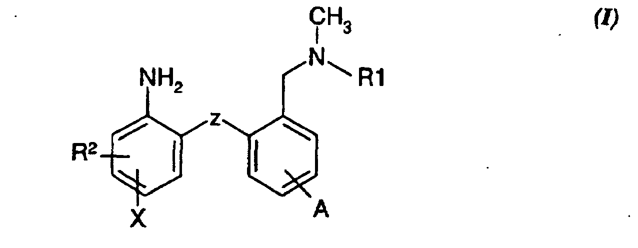
or a pharmaceutically acceptable salt thereof; wherein
X is hydrogen, Cl, Br, I, NO₂, NR³R⁴ or -L-Ch;
Z is S, O, NR⁶, CR⁷R⁸, C(O) or -C(=CR⁷R⁸)-;
A is hydrogen, Cl, I, Br or -L-Ch;
R¹ is hydrogen, C₁-C₅ alkyl, C₃-C₈ cycloalkyl, C₁₋₅ alkylcarbonyl, (C₃-C₈ cycloalkyl)carbonyl, phenyl, benzyl, naphthyl, naphthylmethyl or -L-Ch;
R² is hydrogen or methyl;
R³ and R⁴ are independently hydrogen, hydroxy, C₁-C₅ alkyl, C₃-C₈ cycloalkyl, C₁₋₅ alkylcarbonyl, (C₃-C₈ cycloalkyl)carbonyl, phenyl, benzyl, naphthyl, naphthylmethyl or -L-Ch;
R⁶ is hydrogen, C₁-C₅ alkyl, C₃-C₈ cycloalkyl, C₁₋₅ alkylcarbonyl, (C₃-C₈ cycloalkyl)carbonyl, phenyl, benzyl, naphthyl, or naphthylmethyl or -L-Ch;
R¹ and R⁸ are independently hydrogen, C₁-C₅ alkyl or chloro;
L is a covalent bond or a linking group selected from the group consisting of - (CH₂)ₙ- and -(CH₂)ₙ-C(O)-, where n is 1-5; and
Ch is a tetradentate ligand capable of chelating a metal;
with the proviso that one and only one of A, X, R¹, R³, R⁴, or R⁶ is -L-Ch; or with the proviso that one or both of X or A is Br or I.

2. A compound of claim 1, wherein
X is Br or I;
Z is S, O, NR⁶, CR⁷R⁸, C(O) or -C(=C⁷R⁸)-;
A is hydrogen;
R¹ is hydrogen, C₁-C₅ alkyl, C₃-C₈ cycloalkyl, C₁₋₅ alkylcarbonyl, (C₃-C₈ cycloalkylxarbonyl, phenyl, benzyl, naphthyl or naphthylmethyl;
R² is hydrogen or methyl;
R⁶ is hydrogen, C₁-C₅ alkyl, C₃-C₈ cycloalkyl, C₁₋₅ alkylcarbonyl, (C₃-C₈ cycloalkyl)carbonyl, phenyl, benzyl, naphthyl or naphthylmethyl; and
R⁷ and R⁸ are independently hydrogen, C₁-C₅ alkyl or chloro.

3. A compound of claim 1, wherein
X is hydrogen, Cl, Br, I, NO₂ or NR³R⁴;
Z is S, O NR⁶, CR⁷R⁸, C(O) or -C(=CR⁷R⁸)-;
A is Br or I;
R¹ is hydrogen, C₁-C₅ alkyl, C₃-C₈ cycloalkyl, C₁₋₅ alkylcarbonyl, (C₃-C₈ cycloalkyl)carbonyl, phenyl, benzyl, naphthyl or naphthylmethyl;
R² is hydrogen or methyl;
R³ and R⁴ are independently hydrogen, hydroxy, C₁-C₅ alkyl, C₃-C₈ cycloalkyl, C₁₋₅ alkylcarbonyl, (C₃-C₈ cycloalkyl)carbonyl, phenyl, benzyl, naphthyl or naphthylmethyl;
R⁶ is hydrogen, C₁-C₅ alkyl, C₃-C₈ cycloalkyl, C₁₋₅ alkylcarbonyl, (C₃-C₈ cycloalkyl)carbonyl, phenyl, benzyl, naphthyl or naphthylmethyl; and
R⁷ and R⁸ are independently hydrogen, C₁-C₅ alkyl or chloro.

4. A compound of claim 2, wherein X is a radioactive isotope selected from the group consisting of ¹²³I, ¹³¹I, ¹²⁵I, ⁷⁷Br and ⁷⁶Br.

5. A compound of claim 4, wherein X is ¹²³I.

6. A compound of claim 3, wherein A is a radioactive isotope selected from the group consisting of ¹²³I, ¹³¹I, ¹²⁵I, ⁷⁷Br and ⁷⁶Br.

7. A compound of claim 6, wherein X is ¹²³I.

8. A compound of claim 1, having the general formula: 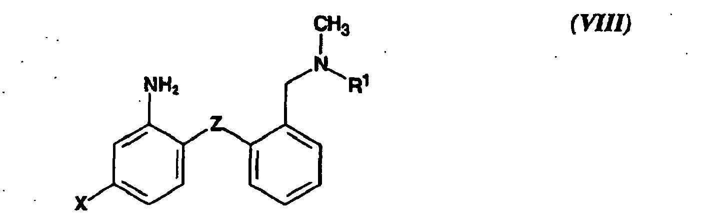 or 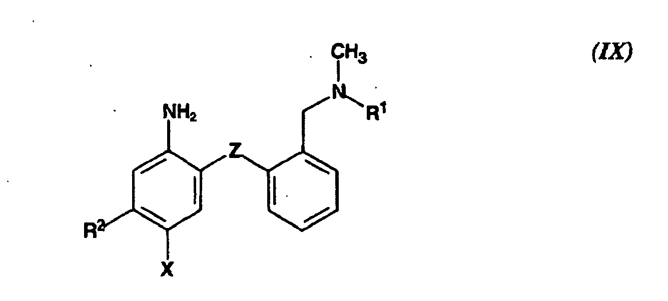 or a pharmaceutically acceptable salt thereof, where X, Z and R¹ and R² are as defined as in claim 1.

9. A compound of claim 8, wherein:
X is I or Br;
Z is S, O, N(CH₃), C(O), C(=CH₂), or CH₂, more preferably S; and
R¹ is methyl.

10. A compound of claim 9, wherein X is ¹²³I, ¹³¹I, ¹²⁵I, ⁷⁷Br or ⁷⁶Br.

11. A compound of claim 8, wherein:
X is hydrogen, I or Br;
Z is S; and
R¹ is hydrogen or methyl.

12. A compound of claim 8, wherein X is ¹²³I, ¹³¹I, ¹²⁵I, ⁷⁷Br or ⁷⁶Br.

13. A compound of claim 8, having the formula VIII, wherein:
X is selected from the group consisting of I, ¹²³I, ¹³¹I, and ¹²⁵I;
Z is -S-; and
R¹ is methyl.

14. A compound of claim 1, wherein Ch is selected from the group consisting of: 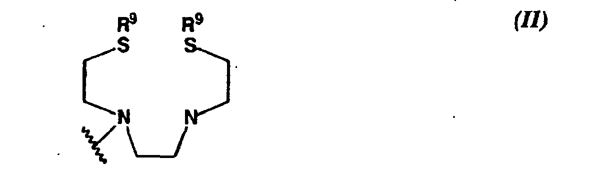 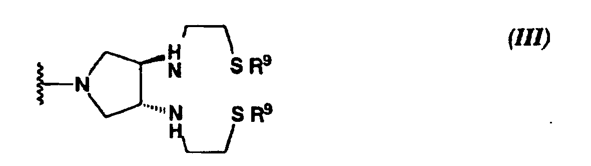 and 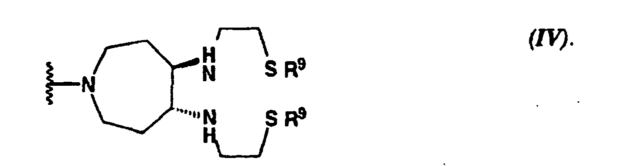
wherein R⁹ is hydrogen or a sulphydryl protecting group.

15. A compound of claim 14, wherein R⁹ is hydrogen, methoxymethyl, methoxyethoxymethyl, *p*-methoxybenzyl or benzyl.

16. A technetium chelate of a compound of claim 1, wherein Ch is selected from the group consisting of 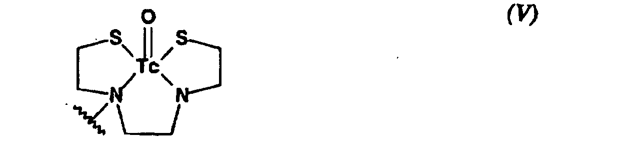 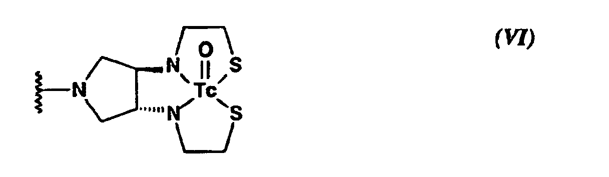 and

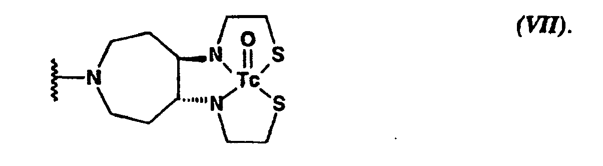

17. A radiopharmaceutical complex, comprising a compound of claim 14, and a radioactive metal selected from the group consisting of technetium-99m, rhenium-186 and rhenium-188 coordinated therewith.

18. A process of radioimagining, comprising:
(i) administering to a mammal an effective amount of a radiopharmaceutical composition, wherein said composition comprises: a radiopharmaceutical compound of claim 4 and a pharmaceutically acceptable carrier or diluent, and
(ii) radioimaging said mammal after allowing sufficient time for said composition to localize in a tissue of said mammal.

19. A process of radioimaging, comprising:
(i) administering to a mammal an effective amount of a radiopharmaceutical composition, wherein said composition comprises: a radiopharmaceutical compound of claim 6 and a pharmaceutically acceptable carrier or diluent, and
(ii) radioimaging said mammal after allowing sufficient time for said composition to localize in a tissue of said mammal.

20. A process of radioimaging, comprising:
(i) administering to a mammal an effective amount of a radiopharmaceutical composition, wherein said composition comprises: a technetium compound of claim 16 and a pharmaceutically acceptable carrier or diluent, and
(ii) radioimaging said mammal after allowing sufficient time for said composition to localize in a tissue of said mammal.

21. A process of radioimaging, comprising:
(i) administering to a mammal an effective amount of a composition, wherein said composition comprises a radiopharmaceutical compound of claim 13 and a pharmaceutically acceptable carrier or diluent, and
(ii) radioimaging said mammal after allowing sufficient time for said composition to localise in a tissue of said mammal.

## Patentansprüche

1. Verbindung nach Formel I: 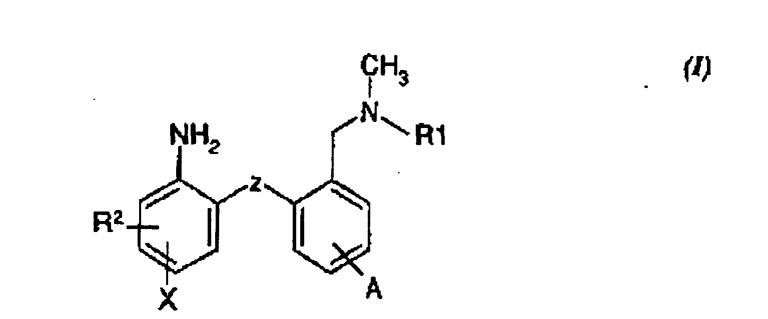
oder ein pharmazeutisch akzeptables Salz derselben, wobei
X Wasserstoff, Cl, Br, I, NO₂, NR³R⁴ oder -L-Ch ist;
Z S, O, NR⁶, CR⁷R⁸, C(O) oder -C(=CR⁷R⁸)- ist;
A Wasserstoff, Cl, I, Br oder -L-Ch ist;
R¹ Wasserstoff, C₁-C₅-Alkyl, C₃-C₈-Cycloalkyl, C₁₋₅-Alkylcarbonyl, (C₃₋C₈-Cycloalkyl)carbonyl, Phenyl, Benzyl, Naphthyl, Naphthylmethyl oder -L-Ch ist;
R² Wasserstoff oder Methyl ist:
R³ und R⁴ unabhängig voneinander Wasserstoff, Hydroxy, C₁-C₅-Alkyl, C₃-C₈-Cycloalkyl, C₁₋₅-Alkylcarbonyl, (C3-C8-Cycloalkyl)carbonyl, Phenyl, Benzyl, Naphthyl, Naphthylmethyl oder -L-Ch sind;
R⁶ Wasserstoff, C₁-C₅-Alkyl, C₃-C₈-Cycloalkyl, C₁₋₅-Alkylcarbonyl, (C₃₋C₈-Cycloalkyl)carbonyl, Phenyl, Benzyl, Naphthyl, Naphthylmethyl oder -L-Ch ist;
R⁷ und R⁸ unabhängig voneinander Wasserstoff, C₁-C₅-Alkyl oder Chlor sind;
L eine kovalente Bindung oder Verbindungsgruppe ist, ausgewählt aus der Gruppe bestehend aus -(CH₂)ₙ- und -(CH₂)ₙ-C(O)-, wo n 1-5 ist, und
Ch ein vierzähliger Ligand ist, der mit einem Metall Chelate bilden kann;
unter dem Vorbehalt, dass eins und auch nur eins von A, X, R¹, R³, R⁴ oder R⁶ ―L-Ch ist; oder unter dem Vorbehalt, dass eins von X oder A oder beide Br oder I sind.

2. Verbindung nach Anspruch 1, **dadurch gekennzeichnet, dass**
X Br oder I ist;
Z S, O, NR⁶, CR⁷R⁸, C(O) oder ―C(=CR⁷R⁸)― ist;
A Wasserstoff ist;
R¹ Wasserstoff, C₁-C₅-Alkyl, C₃-C₈-Cycloalkyl, C₁₋₅-Alkylcarbonyl, (C₃₋C₈-Cycloalkyl)carbonyl, Phenyl, Benzyl, Naphthyl oder Naphthylmethyl ist;
R² Wasserstoff oder Methyl ist;
R⁶ Wasserstoff, C₁-C₅-Alkyl, C₃-C₈-Cycloalkyl, C₁₋₅-Alkylcarbonyl, (C₃₋C₈-Cycloalkyl)carbonyl, Phenyl, Benzyl, Naphthyl oder Naphthylmethyl ist; und
R⁷ und R⁸ unabhängig voneinander Wasserstoff, C₁-C₅-Alkyl oder Chlor sind.

3. Verbindung nach Anspruch 1, **dadurch gekennzeichnet, dass**
X Wasserstoff, Cl, Br, I, NO₂ oder NR³R⁴ ist;
Z S, O, NR⁶, CR⁷R⁸, C(O) oder -C(=CR⁷R⁸)- ist;
A Br oder I ist;
R¹ Wasserstoff, C₁-C₅-Alkyl, C₃-C₈-Cycloalkyl, C₁₋₅-Alkylcarbonyl, (C₃₋C₈-Cycloalkyl)carbonyl, Phenyl, Benzyl, Naphthyl oder Naphthylmethyl ist;
R² Wasserstoff oder Methyl ist;
R³ und R⁴ unabhängig voneinander Wasserstoff, Hydroxy, C₁-C₅-Alkyl, C₃-C₈-Cycloalkyl, C₁₋₅-Alkylcarbonyl, (C3-C8-Cycloalkyl)carbonyl, Phenyl, Benzyl, Naphthyl oder Naphthylmethyl sind;
R⁶ Wasserstoff, C₁-C₅-Alkyl, C₃-C₈-Cycloalkyl, C₁₋₅-Alkylcarbonyl, (C₃₋C₈-Cycloalkyl)carbonyl, Phenyl, Benzyl, Naphthyl oder Naphthylmethyl ist; und
R⁷ und R⁸ unabhängig voneinander Wasserstoff, C₁-C₅-Alkyl oder Chlor sind.

4. Verbindung nach Anspruch 2, bei welcher X ein radioaktives Isotop ist, ausgewählt aus der Gruppe bestehend aus ¹²³I, ¹³¹I, ¹²⁵I, ⁷⁷Br und ⁷⁶Br.

5. Verbindung nach Anspruch 4, **dadurch gekennzeichnet, dass** X ¹²³I ist.

6. Verbindung nach Anspruch 3, **dadurch gekennzeichnet, dass** A ein radioaktives Isotop ist, ausgewählt aus der Gruppe bestehend aus ¹²³I, ¹³¹I, ¹²⁵I, ⁷⁷Br und ⁷⁶Br.

7. Verbindung nach Anspruch 6, **dadurch gekennzeichnet, dass** X ¹²³I ist.

8. Verbindung nach Anspruch 1, das die allgemeine Formel besitzt: 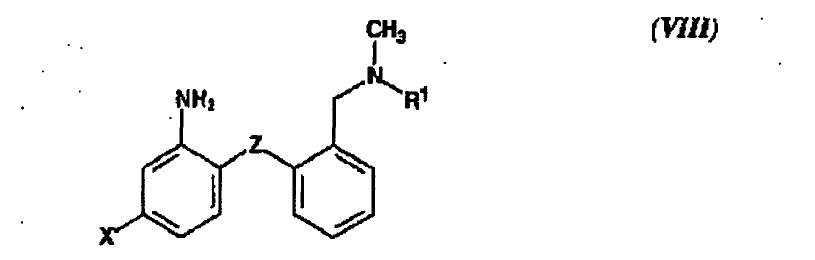 oder 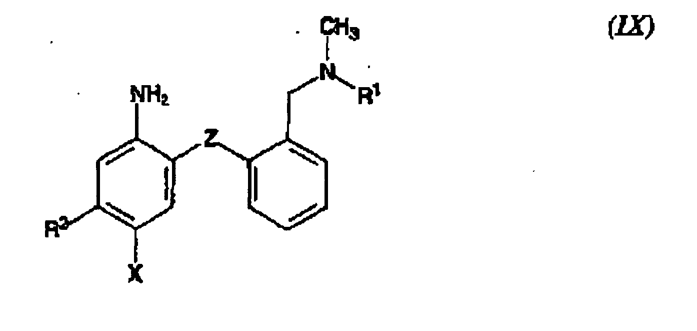
oder ein pharmazeutisch akzeptables Salz derselben, wobei X, Z und R¹ und R² wie in Anspruch 1 definiert sind.

9. Verbindung nach Anspruch 8, bei welcher
X I oder Br ist;
Z S, O, N(CH₃), C(O), C(=CH₂) oder CH₂, insbesondere S ist, und
R¹ Methyl ist.

10. Verbindung nach Anspruch 9, bei welcher X¹²³I, ¹³¹I, ¹²⁵I, ⁷⁷Br oder ⁷⁶Br ist.

11. Verbindung nach Anspruch 8, bei welcher
X Wasserstoff, I oder Br ist;
Z S ist, und
R¹ Wasserstoff oder Methyl ist.

12. Verbindung nach Anspruch 8, bei welcher X ¹²³I, ¹³¹I, ¹²⁵I, ⁷⁷Br oder ⁷⁶Br ist.

13. Verbindung nach Anspruch 8, die die Formel VIII besitzt, wobei
X ausgewählt ist aus der Gruppe bestehend aus ¹²³I, ¹³¹I und ¹²⁵I;
Z -S- ist; und
R¹ Methyl ist.

14. Verbindung nach Anspruch 1, bei welcher Ch ausgewählt ist aus der Gruppe bestehend aus: 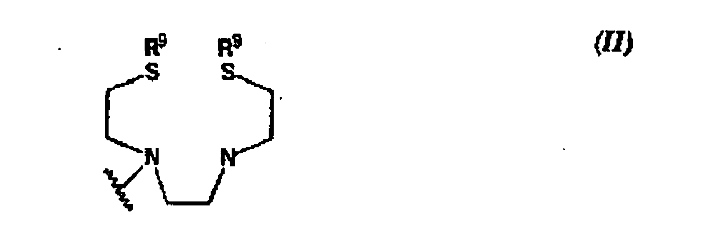 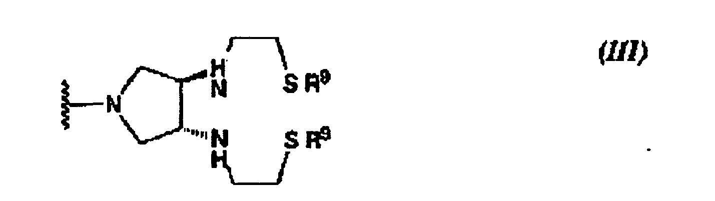 und 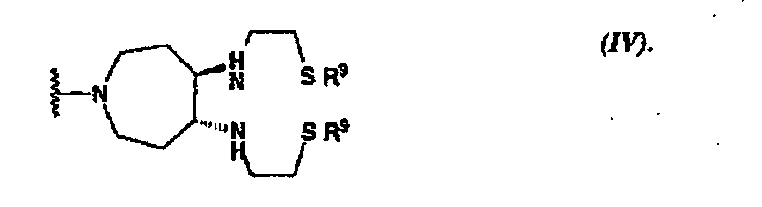
wobei R⁹ Wasserstoff oder eine Sulfhydryl-Schutzgruppe ist.

15. Verbindung nach Anspruch 14, bei welcher R⁹ Wasserstoff, Methoxymethyl, Methoxyethoxymethyl, *p*-Methoxybenzyl oder Benzyl ist.

16. Technetiumchelat einer Verbindung nach Anspruch 1, wobei Ch ausgewählt ist aus der Gruppe bestehend aus 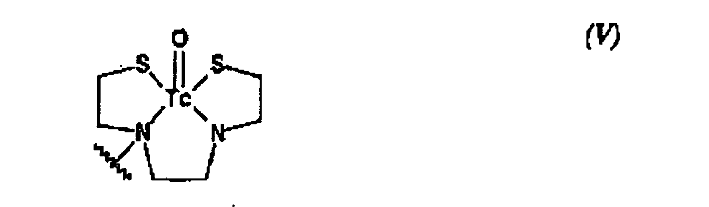 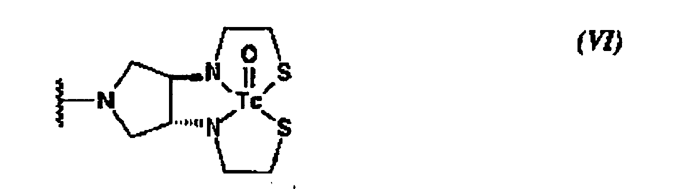
und

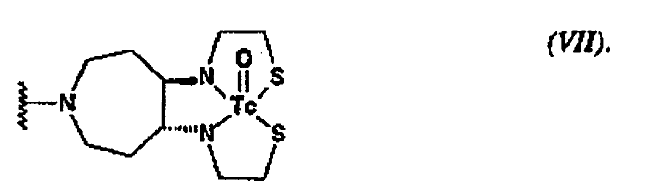

17. Radiopharmazeutischer Komplex, der eine Verbindung nach Anspruch 14 und radioaktives Metall aufweist, das ausgewählt ist aus der Gruppe bestehend aus Technetium-99m, Rhenium-186 und Rhenium-188 und daran koordinativ gebunden ist.

18. Szintigraphieverfahren, welches aufweist:
i) Verabreichung einer wirksamen Menge einer radiopharmazeutischen Zusammensetzung an ein Säugetier, wobei die Zusammensetzung eine radiopharmazeutische Verbindung nach Anspruch 4 und ein(en) pharmazeutisch akzeptablen/es Träger oder Verdünnungsmittel aufweist, und
ii) Szintigraphische Bilderzeugung von dem Säugetier nachdem der Zusammensetzung ausreichend Zeit gegeben wurde, um sich im Gewebe des Säugetiers zu lokalisieren.

19. Szintigraphieverfahren, welches aufweist:
i) Verabreichung einer wirksamen Menge einer radiopharmazeutischen Zusammensetzung an ein Säugetier, wobei die Zusammensetzung eine radiopharmazeutische Verbindung nach Anspruch 6 und ein(en) pharmazeutisch akzeptablen/es Träger oder Verdünnungsmittel aufweist, und
ii) Szintigraphische Bilderzeugung von dem Säugetier nachdem der Zusammensetzung ausreichend Zeit gegeben wurde, um sich im Gewebe des Säugetiers zu lokalisieren.

20. Szintigraphieverfahren, welches aufweist:
i) Verabreichung einer wirksamen Menge einer radiopharmazeutischen Zusammensetzung an ein Säugetier, wobei die Zusammensetzung eine Technetium-Verbindung nach Anspruch 16 und ein(en) pharmazeutisch akzeptablen/es Träger oder Verdünnungsmittel aufweist, und
ii) Szintigraphische Bilderzeugung von dem Säugetier nachdem der Zusammensetzung ausreichend Zeit gegeben wurde, um sich im Gewebe des Säugetiers zu lokalisieren.

21. Szintigraphieverfahren, welches aufweist:
i) Verabreichung einer wirksamen Menge einer radiopharmazeutischen Zusammensetzung an ein Säugetier, wobei die Zusammensetzung eine radiopharmazeutische Verbindung nach Anspruch 13 und ein(en) pharmazeutisch akzeptablen/es Träger oder Verdünnungsmittel aufweist, und
ii) Szintigraphische Bilderzeugung von dem Säugetier nachdem der Zusammensetzung ausreichend Zeit gegeben wurde, um sich im Gewebe des Säugetiers zu lokalisieren.

## Revendications

1. Composé de formule I : 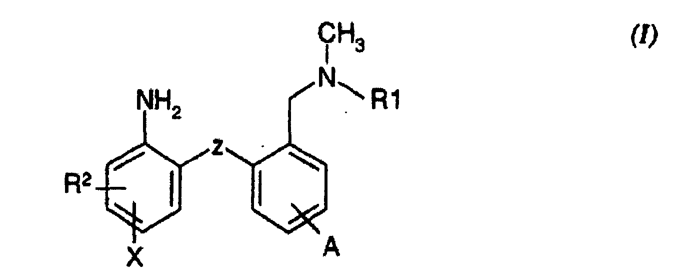
ou sel pharmaceutiquement acceptable de celui-ci ; où
X est hydrogène, Cl, Br, I, NO₂, NR³R⁴ ou -L-Ch ;
Z est S, O, NR⁶, CR⁷R⁸, C(O) ou -C(=CR⁷R⁸)- ;
A est hydrogène, Cl, I, Br ou -L-Ch ;
R¹ est hydrogène, alkyle en C₁-C₅, cycloalkyle en C₃-C₈, (alkyle en C₁-C₅) carbonyle, (cycloalkyle en C₃₋C₈)carbonyle, phényle, benzyle, naphtyle, naphtylméthyle ou -L-Ch;
R² est hydrogène ou méthyle ;
R³ et R⁴ sont indépendamment l'un de l'autre hydrogène, hydroxy, alkyle en C₁-C₅, cycloalkyle en C₃-C₈, (alkyle en C₁-C₅)carbonyle, (cycloalkyle en C₃₋C₈)carbonyle, phényle, benzyle, naphtyle, naphtylméthyle ou -L-Ch;
R⁶ est hydrogène, alkyle en C₁-C₅, cycloalkyle en C₃-C₈, (alkyle en C₁-C₅)carbonyle, (cycloalkyle en C₃₋C₈)carbonyle, phényle, benzyle, naphtyle, naphtylméthyle ou -L-Ch;
R⁷ et R⁸ sont indépendamment l'un de l'autre hydrogène, alkyle en C₁-C₅ ou chloro ;
L est une liaison covalente ou un groupement de liaison choisi dans le groupe constitué de -(CH₂)ₙ- et -(CH₂)ₙ-C(O)-, où n est 1-5 ; et
Ch est un ligand tétradentate capable de chélater un métal ;
à condition qu'un et un seul élément parmi A, X, R¹, R³, R⁴ et R⁶ soit -L-Ch; ou à condition qu'un ou deux éléments parmi X et A soit Br ou I.

2. Composé selon la revendication 1,
**caractérisé en ce que**
X est Br ou I;
Z est S, O, NR⁶, CR⁷R⁸, C(O) ou -C(=CR⁷R⁸)- ;
A est hydrogène;
R¹ est hydrogène, alkyle en C₁-C₅, cycloalkyle en C₃-C₈, (alkyle en C₁-C₅) carbonyle, (cycloalkyle en C₃₋C₈)carbonyle, phényle, benzyle, naphtyle ou naphtylméthyle;
R² est hydrogène ou méthyle ;
R⁶ est hydrogène, alkyle en C₁-C₅, cycloalkyle en C₃-C₈, (alkyle en C₁-C₅)carbonyle, (cycloalkyle en C₃₋C₈)carbonyle, phényle, benzyle, naphtyle ou naphtylméthyle; et
R⁷ et R⁸ sont indépendamment l' un de l'autre hydrogène, alkyle en C₁-C₅ ou chloro.

3. Composé selon la revendication 1,
**caractérisé en ce que**
X est hydrogène, Cl, Br, I, NO₂ ou NR³R⁴ ;
Z est S, O, NR⁶, CR⁷R⁸, C(O) ou -C(=CR⁷R⁸)- ;
A est Br ou I;
R¹ est hydrogène, alkyle en C₁-C₅, cycloalkyle en C₃-C₈, (alkyle en C₁-C₅) carbonyle, (cycloalkyle en C₃₋C₈)carbonyle, phényle, benzyle, naphtyle ou naphtylméthyle;
R² est hydrogène ou méthyle ;
R³ et R⁴ sont indépendamment l'un de l'autre hydrogène, hydroxy, alkyle en C₁-C₅, cycloalkyle en C₃-C₈, (alkyle en C₁-C₅)carbonyle, (cycloalkyle en C₃₋C₈) carbonyle, phényle, benzyle, naphtyle ou naphtylméthyle;
R⁶ est hydrogène, alkyle en C₁-C₅, cycloalkyle en C₃-C₈, (alkyle en C₁-C₅)carbonyle, (cycloalkyle en C₃₋C₈)carbonyle, phényle, benzyle, naphtyle ou naphtylméthyle;
R⁷ et R⁸ sont indépendamment l'un de l' autre hydrogène, alkyle en C₁-C₅ ou chloro.

4. Composé selon la revendication 2, **caractérisé en ce que** X est un isotope radioactif choisi dans le groupe constitué de ¹²³I, ¹³¹I, ¹²⁵I ⁷⁷Br et ⁷⁶Br.

5. Composé selon la revendication 4, **caractérisé en ce que** X est ¹²³I.

6. Composé selon la revendication 3, **caractérisé en ce que** A est un isotope radioactif choisi dans le groupe constitué de ¹²³I, ¹³¹I, ¹²⁵I, ⁷⁷Br et ⁷⁶Br.

7. Composé selon la revendication 6, **caractérisé en ce que** X est ¹²³I,

8. Composé selon la revendication 1 présentant la formule générale : 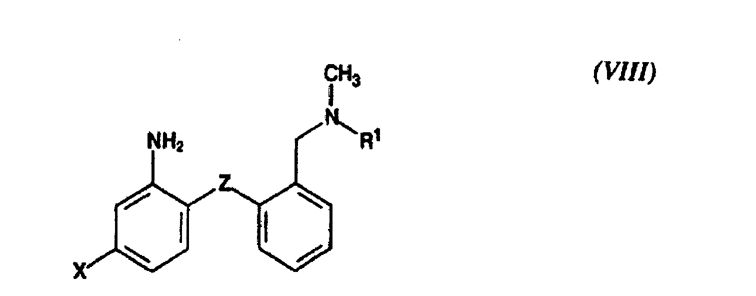 ou 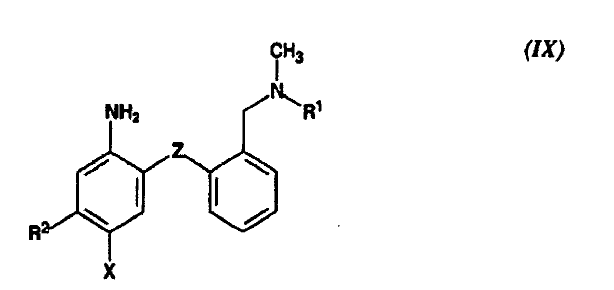
ou sel pharmaceutiquement acceptable de celui-ci, où X, Z et R¹ et R² sont tels que définis à la revendication 1.

9. Composé selon la revendication 8,
**caractérisé en ce que**
X est I ou Br;
Z est S, O, N(CH₃) , C(O) , C(=CH₂) ou CH₂, plus préférablement S ; et
R¹ est méthyle.

10. Composé selon la revendication 9,
**caractérisé en ce que** X est ¹²³I, ¹³¹I, ¹²⁵I, ⁷⁷Br ou ⁷⁶Br.

11. Composé selon la revendication 8,
**caractérisé en ce que** :
X est hydrogène, I ou Br ;
Z est S ; et
R¹ est hydrogène ou méthyle.

12. Composé selon la revendication 8,
**caractérisé en ce que** X est ¹²³I, ¹³¹I, ¹²⁵I, ⁷⁷Br ou ⁷⁶Br.

13. Composé selon la revendication 8, présentant la formule VIII, **caractérisé en ce que**:
X est choisi dans le groupe constitué de I, ¹²³I, ¹³¹I, et ¹²⁵I;
Z est -S- ; et
R¹ est méthyle.

14. Composé selon la revendication 1, **caractérisé en ce que** Ch est choisi dans le groupe constitué de : 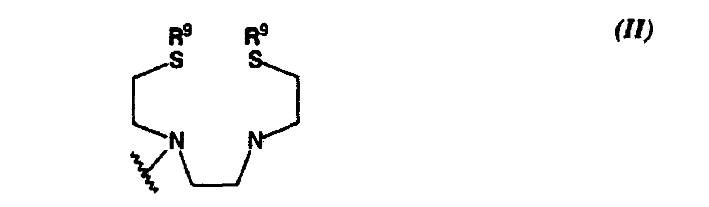 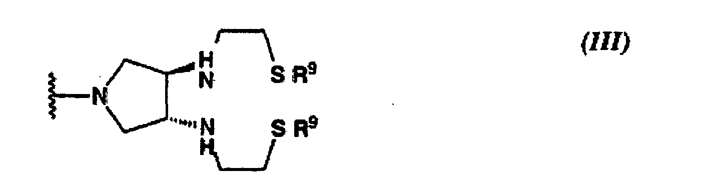 et 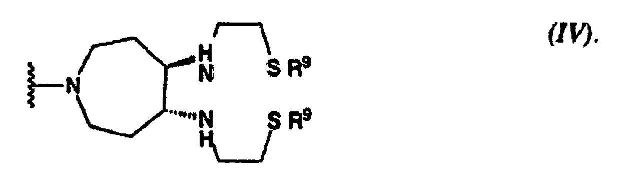
où R⁹ est hydrogène ou un groupement protecteur sulfydryle.

15. Composé selon la revendication 14, **caractérisé en ce que** R⁹ est hydrogène, méthoxyméthyle, méthoxyéthoxyméthyle, p-méthoxybenzyle ou benzyle.

16. Chélate de technétium d'un composé selon la revendication 1, **caractérisé en ce que** Ch est choisi dans le groupe constitué de 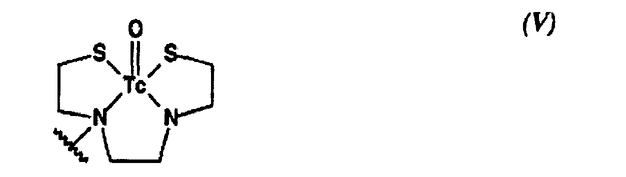 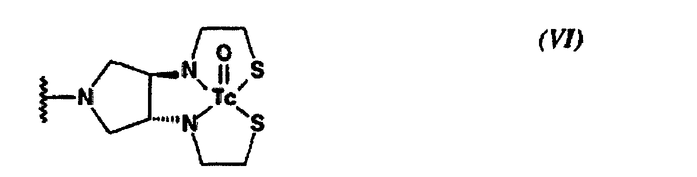 et

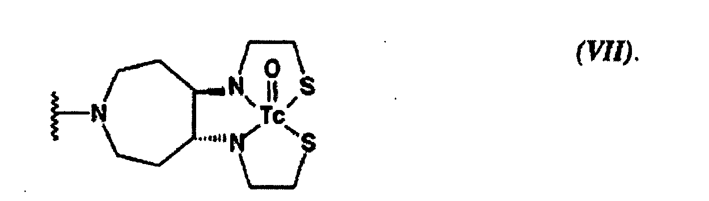

17. Complexe radiopharmaceutique comprenant un composé selon la revendication 14 et un métal radioactif choisi dans le groupe constitué du technétium 99m, du rhénium 186 et du rhénium 188 coordonné à celui-ci.

18. Procédé de radio-imagerie, comprenant :
(i) l'administration à un mammifère d'une quantité efficace d'une composition radiopharmaceutique, ladite composition comprenant un composé radiopharmaceutique selon la revendication 4 et un excipient ou diluant pharmaceutiquement acceptable, et
(ii) la radio-imagerie dudit mammifère après avoir laissé suffisamment de temps à ladite composition pour se localiser dans un tissu dudit mammifère.

19. Procédé de radio-imagerie, comprenant:
(i) l'administration à un mammifère d'une quantité efficace d'une composition radiopharmaceutique, ladite composition comprenant un composé radiopharmaceutique selon la revendication 6 et un excipient ou diluant pharmaceutiquement acceptable, et
(ii) la radio-imagerie dudit mammifère après avoir laissé suffisamment de temps à ladite composition pour se localiser dans un tissu dudit mammifère.

20. Procédé de radio-imagerie, comprenant:
(i) l'administration à un mammifère d'une quantité efficace d'une composition radiopharmaceutique, ladite composition comprenant un composé de technétium selon la revendication 16 et un excipient ou diluant pharmaceutiquement acceptable, et
(ii) la radio-imagerie dudit mammifère après avoir laissé suffisamment de temps à ladite composition pour se localiser dans un tissu dudit mammifère.

21. Procédé de radio-imagerie, comprenant:
(i) l'administration à un mammifère d'une quantité efficace d'une composition, ladite composition comprenant un composé radiopharmaceutique selon la revendication 13 et un excipient ou diluant pharmaceutiquement acceptable, et
(ii) la radio-imagerie dudit mammifère après avoir laissé suffisamment de temps à ladite composition pour se localiser dans un tissu dudit mammifère.
